# EUROPÄISCHE PATENTSCHRIFT

(11) **EP 3 773 151 B1**
(45) Veröffentlichungstag und Bekanntmachung des Hinweises auf die Patenterteilung: **11.11.2026**
(21) Anmeldenummer: 18749297.0
(22) Anmeldetag: 12.07.2018
(51) Int. Cl.: A61B 3/18, A61B 3/16, A61B 3/12

(54) **VORRICHTUNG ZUR BESTIMMUNG RETINALER BLUTDRUCKWERTE UND ZUM MAPPING RETINALER BLUTDRUCKWERTE UND PERFUSIONSDRUCKWERTE**
DEVICE FOR DETERMINING RETINAL BLOOD PRESSURE VALUES AND FOR MAPPING RETINAL BLOOD PRESSURE VALUES AND PERFUSION PRESSURE VALUES
DISPOSITIF PERMETTANT DE DÉTERMINER DES VALEURS DE PRESSION ARTÉRIELLE RÉTINIENNES ET DE MAPPER DES VALEURS DE PRESSION ARTÉRIELLE RÉTINIENNES ET DES VALEURS DE PRESSION DE PERFUSION RÉTINIENNES

(30) Priorität: 29.03.2018 DE 102018107622
(43) Veröffentlichungstag der Anmeldung: 17.02.2021
(73) Patentinhaber: iMEDOS Health GmbH, 07745 Jena (DE)
(72) Erfinder: VILSER, Walthard, 07407 Rudolstadt (DE); KRAUß, Benedikt, 07745 Jena (DE); RIEMER, Thomas, 07751 Jena (DE)
(74) Vertreter: Gleim Petri Patent- und Rechtsanwaltspartnerschaft mbB
(86) Internationale Anmeldenummer: PCT/DE2018/100638
(87) Internationale Veröffentlichungsnummer: WO 2019/185073

(56) Entgegenhaltungen:
- WO-A1-2015/131236
- WO-A2-2006/091811
- WO-A2-2017/035406
- S. MOJTABA GOLZAN ET AL: "Dynamic Association between Intraocular Pressure and Spontaneous Pulsations of Retinal Veins", CURRENT EYE RESEARCH, vol. 36, no. 1, 21 January 2011 (2011-01-21), US, pages 53 - 59, XP055533102, ISSN: 0271-3683, DOI: 10.3109/02713683.2010.530731
- WILLIAM H. MORGAN ET AL: "Retinal venous pulsation: Expanding our understanding and use of this enigmatic phenomenon", PROGRESS IN RETINAL AND EYE RESEARCH, vol. 55, 1 November 2016 (2016-11-01), GB, pages 82 - 107, XP055532942, ISSN: 1350-9462, DOI: 10.1016/j.preteyeres.2016.06.003

## Beschreibung

Die Erfindung betrifft eine Vorrichtung und ein Verfahren, mit denen im Auge eines Patienten durch künstliches Einbringen eines veränderbaren Stimulationsdruckes SD der Intraokulardruck IOP erhöht wird, der das Eintreten charakteristischer Messkriterien in der Netzhaut des Auges bewirkt, die eine Ableitung globaler und lokaler retinaler Blutdruckwerte rP aus dem Intraokulardruckwert IOP erlauben. Ein gattungsgemäßes Verfahren und eine gattungsgemäße Vorrichtung sind aus der DE 195 14 796 C1 bekannt.

Die Erfindung betrifft auch eine Vorrichtung und ein Verfahren zum Mapping retinaler Blutdruckwerte rP und Perfusionsdruckwerte rPP im Sehnervenkopf (globale retinale Blutdruckwerte rP) und an verschiedenen Orten der Netzhaut (lokale retinale Blutdruckwerte rP) auf einem Bild oder mehreren Bilden der Netzhaut.

Dass Änderungen des retinalen Perfusionsdruckes rPP wesentlich den retinalen Blutflussbestimmen und eine entscheidende Rolle für die Entstehung verschiedener okulärer Erkrankungen spielen, ist in der modernen Medizin bekannt. Berechnet wird der retinale Perfusionsdruck rPP über die Differenz des retinalen arteriellen Blutdrucks rPa des in das Auge einströmenden Blutes und des retinalen venösen Blutdrucks rPv des aus dem Auge ausströmenden Blutes. $rPP = {rP}_{a} - {rP}_{v}$

Der retinale venöse Blutdruck rPv ist dabei stark abhängig von den äußeren Gegebenheiten und nimmt entweder den Wert des Intraokulardrucks IOP an, wenn der Intraokulardruck IOP größer ist als der retinale venöse Blutdruck außerhalb des Augapfels RVP (häufig auch als retinaler venöser Abflussdruck bezeichnet), oder ist gleich dem retinalen venösen Blutdruck außerhalb des Augapfels RVP, insofern dieser größer ist als der Intraokulardruck IOP. In Sonderfällen kann der retinale venöse Blutdruck rPv auch den Wert des Intrakranialdrucks annehmen. Dies gilt jedoch nur, wenn der Intrakranialdruck größer als der Intraokulardruck IOP und der retinale venöse Blutdruck außerhalb des Augapfels RVP ist.

Die Ophthalmodynamometrie ist eine Methode zur Bestimmung globaler retinaler Blutdruckwerte rP anhand der bekannten visuell sichtbaren Pulsphänomene bei Erreichung der retinalen Blutdruckwerte rP. Zur retinalen Blutdruckmessung (Ophthalmodynamometrie) wird der Intraokulardruck IOP erhöht. In Anlehnung an die Blutdruckmessung am Oberarm fährt man zu Beginn der Messung den Intraokulardruck IOP zunächst auf suprasystolische arterielle Blutdruckwerte, größer dem retinalen arteriellen systolischen Blutdruckwert rP_{asys}, hoch, die erreicht sind, wenn man keine Gefäßpulsation sehen kann, und senkt anschließend den Intraokulardruck IOP langsam ab. Die ersten sichtbaren arteriellen Gefäßpulsationen sind das visuelle Messkriterium für das Erreichen des globalen retinalen arteriellen systolischen Blutdruckes rP_{asys} in der Netzhaut. Sieht man die arteriellen Gefäßpulsationen wieder verschwinden (weiteres visuelles Messkriterium), hat der Intraokulardruck IOP den retinalen arteriellen diastolischen Blutdruck rP_{adia} in der Netzhaut erreicht. In den letzten Jahren wurde die Ophthalmodynamometrie zunehmend benutzt, um den retinalen venösen Blutdruck innerhalb des Augapfels rPv zu messen. Als visuelles Messkriterium benutzt man den sogenannten Venenkollaps im Bereich des Sehnervenkopfes. Der Venenkollaps tritt im Bereich des Sehnervenkopfes dort auf, wo die Netzhautvenen den Augapfel gerade verlassen, solange der Intraokulardruck IOP größer als der retinale venöse Blutdruck außerhalb des Augapfels RVP ist. Ist der retinale venöse Blutdruck außerhalb des Augapfels RVP höher als der Intraokulardruck IOP, dann fehlt der spontane Venenkollaps. Um den retinalen venösen Blutdruck außerhalb des Augapfels RVP zu messen, erhöht man den Intraokulardruck IOP solange, bis der spontane Venenkollaps gerade zu sehen ist. Der dazu gehörige Intraokulardruck IOP entspricht dann dem retinalen venösen Blutdruck außerhalb des Augapfels RVP.

Ein bekanntes und in der Medizin angewandtes Gerät zur Durchführung der Ophthalmodynamometrie wird in der vorgenannten DE 195 14 796 C1 beschrieben. Hierbei wird ein Kontaktglas durch den Untersucher auf die Hornhaut des Auges aufgesetzt. Über das Kontaktglas beobachtet der Untersucher den Sehnervenkopf des Auges und erhöht dabei den Druck mit dem Kontaktglas auf das Auge und damit den Intraokulardruck IOP im Auge, bis die visuellen Messkriterien zu sehen sind. Die Kraft, mit dem das Kontaktglas auf das Auge drückt, wird mit einem Kontaktglasdynamometer gemessen und angezeigt. Aus dieser Kraft lässt sich dann der zu dem jeweiligen visuellen Messkriterium gehörende Intraokulardruckwert IOP bzw. retinale Blutdruckwert rP berechnen.

Um aus dem gemessenen Druck, mit dem der Untersucher das Kontaktglasdynamometer auf das Patientenauge drückt, den Intraokulardruck IOP genau ableiten zu können, ist eine Eichung des Gerätes notwendig, welche in regelmäßigen Abständen wiederholt werden muss. Bei der Eichung wird von einem "Standardauge" ausgegangen, das heißt, das Auftreten unterschiedlicher Augenformen sowie verschiedener okulärer Gewebsparameter wird nicht berücksichtigt. Auch die manuelle Kraft- bzw. Druckeinleitung auf das Auge birgt ein erhebliches Fehlerpotential.

Ein weiteres Gerät zur Durchführung der Ophthalmodynamometrie wird in der DE 35 11 938 A1 beschrieben. In diesem Fall wird die künstliche Erhöhung des Intraokulardrucks IOP mit Hilfe der Saugnapf-Kompressions-Tonometrie realisiert, indem manuell ein Saugnapf durch einen Unterdruck temporal auf den Augapfel des Patienten fixiert und im Weiteren der Unterdruck im Inneren des Saugnapfes erhöht wird. Durch diese Erhöhung des Unterdrucks im Saugnapf wird der Augapfel des Patienten zunehmend stärker in den Saugnapf eingesogen. Die dadurch hervorgerufene Verformung des Augapfels führt zu einem Anstieg des Intraokulardrucks IOP. Über den direkten Kontakt zum Auge des Patienten werden mit Hilfe einer Wandlereinheit, welche pneumatisch mit dem Saugnapf verbunden ist, die okulären Pulsationen registriert und aufgezeichnet. Über die Auswertung der dabei aufgezeichneten Kurven und den geeichten Zusammenhang zwischen dem Unterdruck im Inneren des Saugnapfes und dem Intraokulardruck IOP können die verschiedenen retinalen Blutdruckwerte rP bestimmt werden. Hierzu zählen der retinale arterielle systolische Blutdruck rP_{asys}, der ziliare arterielle systolische Blutdruck und der okuläre arterielle diastolische Blutdruck. Darüber hinaus können noch eine Reihe weiterer das Auge betreffender Parameter, wie zum Beispiel die okuläre Perfusion, die autoregulatorische Kapazität und der kritische Punkt, bei dem das okuläre Pulsblutvolumen abfällt, bestimmt werden.

Auch das in der vorbenannten DE 35 11 938 A1 offenbarte Gerät muss aufgrund der direkten Bestimmung der Erhöhung des Intraokulardrucks IOP aus dem Unterdruck im Inneren des Saugnapfes regelmäßig einer Eichung unterzogen werden. Zudem lässt sich der retinale venöse Blutdruck außerhalb des Augapfels RVP mit dieser Methodik nur bedingt messen, da der Intraokulardruck IOP durch den Unterdruck zur Fixierung des Saugnapfes bereits angehoben wird. Da der retinale venöse Blutdruck außerhalb des Augapfels RVP oft im Bereich des Intraokulardrucks IOP oder leicht darüber liegt, kann diese zur Fixation des Saugnapfes erforderliche Erhöhung des Intraokulardrucks IOP den Anfangspunkt des spontanen Venenkollaps bereits wesentlich überschreiten und so eine Messung des retinalen venösen Blutdruckes außerhalb des Augapfels RVP unmöglich machen. Darüber hinaus findet während der Untersuchung keine Beobachtung der Netzhaut statt, wodurch eine Auswertung nur rein über die okulären Pulsationskurven möglich ist und infrage steht, ob die mit dem Saugnapfverfahren messbaren retinalen Blutdrücke rP mit den globalen eingangs definierten retinalen Blutdruckwerten rP identisch sind.

Die beiden beschriebenen Vorrichtungen sind ursprünglich nicht zur Messung des retinalen venösen Blutdrucks außerhalb des Augapfels RVP vorgesehen, sondern dienen der Messung globaler retinaler arterieller Blutdrücke rPa, welche zum Teil zur Berechnung weiterer okulärer Parameter genutzt werden können.

Eine andere Anwendung ist die Messung des Intrakranialdrucks. Dies ist jedoch nur möglich, wenn der Intrakranialdruck größer als der Intraokulardruck IOP und der retinale venöse Blutdruck außerhalb des Augapfels RVP ist. In diesem Fall nimmt der retinale venöse Blutdruck rPv bzw. auch der retinale venöse Blutdruck außerhalb des Augapfels RVP den Wert des Intrakranialdrucks an. Der Intrakranialdruck kann in diesem Fall über die Messung des retinalen venösen Blutdruckes außerhalb des Augapfels RVP bestimmt werden. Als Untersuchungskriterien dienen wiederum der spontane Venenkollaps oder andere ableitbare Messkriterien, je nach eingesetztem Messverfahren und abgeleitetem Messkriterium. Geräte, welche diese Methodik anwenden, sind zum Beispiel in der EP 2 567 656 B1, der US 2015/0265172 A1 und der DE 10 55 175 B beschrieben.

WO 2006/091811 A2 offenbart eine Vorrichtung zur nicht-invasiven Bestimmung des Intrakranialdrucks (ICP) mit:
- Bildgebungseinheit
- Druckerzeugungseinheit
- Druckmesser
- Steuereinheit

Diese Vorrichtung misst den Ruhe-Intraokulardruck mittels eines Tonometers ohne anliegenden Stimulationsdruck. Der Intraokulardruck während des angelegten Stimulationsdrucks wird mittels einer Kalibrierungskurve oder eines Algorithmus berechnet.

WO 2015/131236 A1 offenbart ein System zur ICP-Bestimmung mit Video-Dynamometer (mit Kontaktlinse) und separatem Tonometer zur Basis-IOP-Messung. Der IOP während des Stimulationsdrucks wird berechnet aus: Basis-IOP, applizierter Kraft (gemessen durch ring force transducer) und Kalibrierung mittels Blutdruckmessung. Es gibt keine direkte IOP-Messung während Stimulationsdruck.

Grundsätzlich lässt sich jedoch sagen, dass all die hier beschriebenen Geräte lediglich zur Bestimmung globaler retinaler Blutdruckwerte rP geeignet sind und somit auch nur die Bestimmung eines globalen retinalen Perfusionsdruckwertes rPP möglich ist und dass wesentliche Fehlerquellen, insbesondere durch manuelle Kraft- bzw. Druckeinleitung über die Eichbeziehungen, das Untersuchungsergebnis verfälschen.

Häufig kann es klinisch sinnvoll oder erforderlich sein, nicht nur die globalen retinalen Blutdrücke rP zu kennen, sondern lokale retinale Blutdrücke rP zu messen und bezogen auf ihren Ort an der Netzhaut zu bewerten, wie z. B. im Falle arterieller und venöser Astverschlüsse. Weiterhin kann es erforderlich sein, lokale retinale Perfusionsdrücke rPP einzelner Kapillarbereiche und größerer Gefäßbereiche (Gefäßnetze) zu untersuchen und im Hinblick auf prädiktive Aussagen oder eine echte Früherkennung gefährdeter Perfusionsdruckverhältnisse und -bereiche zu bewerten und zu erkennen.

Es ist die Aufgabe der Erfindung, eine Vorrichtung und ein Verfahren zu schaffen, mit denen globale und lokale retinale Blutdruckwerte rPmanuell, aber vorteilhaft auch automatisch bestimmt werden können. Die retinalen Blutdruckwerte rP sollen einem Messort oder einem mehrere Messorte umfassenden Messbereich auf der Netzhaut bzw. dem Sehnervenkopf zugeordnet werden können. Vorteilhaft sollen aus den retinalen Blutdruckwerten rP Perfusionsdruckwerte rPP bestimmt und dargestellt werden.

Eine weitere Aufgabe der Erfindung besteht darin, die individuelle Reproduzierbarkeit der Messergebnisse deutlich zu erhöhen, den Einfluss der individuellen Besonderheiten des Augapfels weitestgehend auszuschließen und die Fehlerquellen manueller Druckeinleitung deutlich zu reduzieren und die Messergebnisse vor allem unabhängig von den Besonderheiten des individuellen Auges zu machen.

Das Wesen der Erfindung besteht einerseits darin, dass neben den bekannten weiter oben beschriebenen visuellen Messkriterien zur Bestimmung der globalen retinalen Blutdruckwerte rP weitere subjektive, aber auch objektive lokale Pulsphänomene, Signale und Messkriterien vom Gefäßsystem der Netzhaut und des Sehnervenkopfes genutzt bzw. gebildet oder abgeleitet werden, denen erfindungsgemäß lokal und physiologisch definierbare retinale Blutdruckwerte rP zugeordnet werden können. Vorteilhaft werden die Blutdruckwerte rP und / oder Perfusionsdruckwerte rPP örtlich zugeordnet in einem Druckmappingbild oder örtlich und zeitlich zugeordnet in einer Folge von Druckmappingbildern, deren Hintergrund ein Bild der Netzhaut ist, dargestellt. Erfindungsgemäß können die Untersuchungen weiterhin manuell, aber auch als automatische Messungen ausgeführt werden.

Andererseits werden erfindungsgemäß die Vorrichtung und das Verfahren derart aufgebaut, dass die gemessenen retinalen Blutdrücke rP nicht über unsichere globale geeichte Zusammenhänge zwischen dem veränderbaren Stimulationsdruck SD und dem Intraokulardruck IOP bestimmt werden, sondern direkt mit dem Tonometer am individuellen Patientenauge gemessen oder aus einer individuellen Beziehung eines direkt am Patientenauge bestimmten Zusammenhangs zwischen dem veränderbaren Stimulationsdruck SD und dem Intraokulardruck IOP berechnet werden. Zentrales Element ist dabei eine Einheit zur Erzeugung und Applikation eines veränderbaren Stimulationsdruckes, deren druckvermittelnde Einheit, ein Druckapplikator, am Kopf des Auges fixiert ist und seitlich (außerhalb der Hornhaut) einen veränderbaren Stimulationsdruck SD nasal auf den Augapfel gerichtet einleitet, wobei der Zusammenhang zwischen dem veränderbaren Stimulationsdruck SD und dem Intraokulardruck IOP nicht exakt bekannt ist oder direkt an diesem Auge für dieses Auge ermittelt wurde. Mit dieser Anordnung des Druckapplikators seitlich zum Auge bleibt der Einblick in das Auge für verschiedene Geräte frei, und auch Tonometermessungen am betreffenden Auge sind mit verschiedenen Geräten durchführbar.

Beide beschriebenen erfindungsgemäßen Schwerpunkte werden vorteilhafterweise gemeinsam ausgeführt, können erfindungsgemäß aber auch selbstständig als erfindungsgemäße Lösungen zum Einsatz kommen.

Die Aufgabe der Erfindung wird für eine Vorrichtung zur Bestimmung globaler und lokaler retinaler Blutdruckwerte rP an einem Auge eines Patienten, enthaltend eine auf das Auge wirkende Einheit zur Erzeugung und Applikation eines veränderbaren Stimulationsdruckes und eine Bildgebungseinheit, dadurch gelöst, dass ein Tonometer vorhanden ist, um einen Intraokulardruck IOP im Auge, der sich in Abhängigkeit von einem applizierten veränderbaren Stimulationsdruck SD ändert, zu messen, eine Rechen- und Steuereinheit mit einer Ein- und Ausgabeeinheit vorhanden ist, die mit der Einheit zur Erzeugung und Applikation eines veränderbaren Stimulationsdruckes verbunden ist, und die Einheit zur Erzeugung und Applikation eines veränderbaren Stimulationsdruckes einen Druckapplikator enthält, der am Kopf des Patienten ortsfest zum Auge außerhalb der Hornhaut und eines Lichtweges der Bildgebungseinheit druckfrei flächig an das Auge anlegbar ist.

Vorteilhaft ist die Einheit zur Erzeugung und Applikation eines veränderbaren Stimulationsdruckes so ansteuerbar, dass der applizierte veränderbare Stimulationsdruck SD in Richtung und Geschwindigkeit eines Anstieges änderbar ist sowie konstant gehalten werden kann, und die Ein- und Ausgabeeinheit ist dazu ausgelegt, dass die Ansteuerung durch einen Untersucher über die Ein- und Ausgabeeinheit erfolgt.

Es ist von Vorteil, wenn die Bildgebungseinheit einen digitalen Bildsensor oder eine bildgebende Einheit auf Basis der optischen Kohärenztomografie oder der Laser-Scanning-Technik aufweist und ein digitaler Videorekorder vorhanden ist, der mit der Bildgebungseinheit und der Rechen- und Steuereinheit verbunden ist, und die Ein- und Ausgabeeinheit einen Monitor aufweist und dazu ausgelegt ist, dass der Untersucher wahlweise online Bilder der Bildgebungseinheit oder vom digitalen Videorekorder aufgezeichnete Videosequenzen der Bilder ansehen und zur Untersuchung einsetzen kann.

Vorzugsweise ist eine Daten- und Bildauswerteeinheit vorhanden, die mit dem digitalen Videorekorder, der Bildgebungseinheit, der Rechen- und Steuereinheit und der Ein- und Ausgabeeinheit in Verbindung steht, und die Ein- und Ausgabeeinheit ist dazu ausgelegt ist, dass der Untersucher in am Monitor angezeigten Bildern der Bildgebungseinheit oder Bildern der Videosequenzen des digitalen Videorekorders Messorte für erkannte visuelle Messkriterien festlegen kann und die Koordinaten der Messorte gemeinsam mit den visuellen Messkriterien, jeweils einem retinalen Blutdruckwert rP zugeordnet, abspeichern und in einem Druckmappingbild eintragen kann.

Die Einheit zur Erzeugung und Applikation eines veränderbaren Stimulationsdruckes enthält vorteilhafterweise einen Drucksensor, um jeweils einem gemessenen Intraokulardruckwert IOP bzw. jedem Bild der Videosequenz einen veränderbaren Stimulationsdruckwert SD zuordnen zu können.

Es ist ferner von Vorteil, wenn die Bildgebungseinheit einen digitalen Bildsensor oder eine bildgebende Einheit auf Basis der optischen Kohärenztomografie oder der Laser-Scanning-Technik zur Erzeugung einer Videosequenz aufweist und eine Daten- und Bildauswerteeinheit sowie eine Signalanalyseeinheit vorhanden sind, wobei die Daten- und Bildauswerteeinheit dazu ausgelegt ist, eine bewegungskorrigierte Videosequenz von der Netzhaut des Auges zu erzeugen, und für jedes Pixel oder für eine beliebige Pixelgeometrie von Bildern der Videosequenz ein Signal bildet und einem Zeitsignal der Rechen- und Steuereinheit zuordnet.

Es ist außerdem vorteilhaft, wenn die Bildgebungseinheit eine modifizierte Netzhautkamera mit mindestens zwei Farbkanälen und einem doppelbandigen Bandpassfilter ist und eine Einheit zur Bildung von spektralen Quotientensignalen vorhanden ist, die für jedes Pixel oder eine zusammenfassende Pixelgeometrie der Bilder der Videosequenz ein beleuchtungsunabhängiges spektrales Quotientensignal bildet und es einem Zeitsignal der Rechen- und Steuereinheit zuordnet.

Vorzugsweise ist eine Einheit zur Bildung von Gefäßdurchmessersignalen vorhanden, die für jedes Gefäßsegment ein mit einem Durchmesser korrelierendes Gefäßdurchmessersignal bildet und dieses einem Zeitsignal der Rechen- und Steuereinheit zugeordnet.

Ferner ist das Tonometer bevorzugt ein automatisch messendes Rebound-Tonometer oder Non-Contact-Tonometer und in der Bildgebungseinheit integriert.

Die Aufgabe der Erfindung wird ferner für ein Verfahren zur Bestimmung globaler und lokaler retinaler Blutdruckwerte an einem Auge eines Patienten, bei dem ein veränderbarer Stimulationsdruck SD auf das Auge eingeleitet wird, der zu einer Veränderung des Intraokulardrucks IOP im Auge führt. Parallel dazu wird die Netzhaut beobachtet wird und / oder es wird eine Videosequenz von Bildern der Netzhaut aufgenommen. Dabei wird ein momentaner Intraokulardruckwert IOP einem der retinalen Blutdruckwerte rP dann gleichgesetzt, wenn die Erfüllung eines für diesen retinalen Blutdruckwert rP charakteristischen Messkriteriums an der Netzhaut beobachtet oder aus den Bildern abgeleitet wird. Zu wenigstens einem Zeitpunkt der Erfüllung wird der veränderbare Stimulationsdruck SD über eine Zeitspanne konstant gehalten. Während dieser Zeitspanne erfolgt manuell oder automatisch eine direkte Messung des Intraokulardruckes IOP mit einem Tonometer und der gemessene Intraokulardruckwert IOP wird demjenigen der retinalen Blutdruckwerte rP gleichgesetzt, für den das charakteristische Merkmal erfüllt wurde. Es ist ferner von Vorteil, wenn ein Zeitsignal gebildet wird, dem die gemessenen Intraokulardruckwerte IOP, Stimulationsdruckwerte SD, Bilder und abgeleitete Bilder der Videosequenz sowie Zeitpunkte des Auftretens der charakteristischen Messkriterien und die zugehörigen retinalen Blutdruckwerte rP zugeordnet werden.

Bevorzugt wird aus mindestens zwei direkt gemessenen Intraokulardruckwerten IOP und den über das Zeitsignal jeweils zugeordneten Stimulationsdruckwerten SD der für das individuelle Auge zutreffende individuelle Zusammenhang zwischen dem Intraokulardruck IOP und dem veränderbaren Stimulationsdruck SD berechnet, wobei im Falle nur eines bei Detektion eines der charakteristischen Messkriterien direkt gemessenen Intraokulardruckwertes IOP ein weiterer Intraokulardruckwert IOP ohne Detektion eines der charakteristischen Messkriterien zu einem beliebigen Zeitpunkt erhöhter Stimulationsdruckwerte SD direkt gemessen wird.

Vorzugsweise leitet ein Untersucher während der Aufnahme der Videosequenz online aus den Bildern das Auftreten der charakteristischen Messkriterien globaler retinaler Blutdruckwerte rP ab. Später markiert er offline anhand der Videosequenz interaktiv in den Bildern das Auftreten der charakteristischen Messkriterien lokaler retinaler Blutdrücke rP an Messorten örtlich und zeitlich, bestimmt über das Zeitsignal jeweils die zugehörigen Intraokulardruckwerte IOP, setzt diese jeweils einem retinalen Blutdruckwert rP gleich, speichert die retinalen Blutdruckwerte rP und zugehörigen Messorte und trägt sie in einem Druckmappingbild ein.

Dabei ist es von Vorteil, wenn aus den Bildern der Videosequenz weitere Gefäßdurchmessersignale abgeleitet und jeweils einem Zeitpunkt und einem Gefäßsegment oder einem Gefäßsegmente umfassenden Gefäßabschnitt zugeordnet werden.

Ferner ist es vorteilhaft, wenn aus den Bildern der Videosequenz beleuchtungsunabhängige spektral normierte Signale abgeleitet und einem Zeitpunkt und einem Messort zugeordnet werden.

Den Signalen werden vorzugsweise der Anstieg und Abfall von Gefäßpulsationen oder pulsatorische und kontinuierliche Abblassungen oder Signaländerungen als weitere charakteristische Messkriterien und / oder globale oder lokale retinale Blutdrücke rP als weitere Schwellenwerte zugeordnet und die weiteren charakteristischen Messkriterien und / oder weiteren Schwellenwerte werden zur automatischen Messung bzw. Bestimmung der Intraokulardruckwerte IOP verwendet.

Bevorzugt werden die charakteristischen Messkriterien über die gesamte Netzhaut erfasst und es werden daraus Netzhautbereiche abgeleitet, die pathologische Gefäßbereiche darstellen, welche bei der Analyse des vaskulären Risikos von lokalen retinalen Durchblutungsstörungen besonders beachtet werden können.

Dabei ist es von Vorteil, wenn Messorte oder Gefäßsegmente, bei denen zur gleichen Zeit die gleichen Messkriterien auftreten, zu Gefäßabschnitten oder Gefäßbereichen zusammengefasst und in einem Druckmappingbild zusammengestellt werden und wenn verschiedene retinale Blutdruckwerte rP und / oder Messkriterien farblich kodiert präsentiert werden.

Ferner ist es vorteilhaft, wenn aus lokalen retinalen arteriellen Blutdruckwerten rPa näherungsweise lokale retinale Perfusionsdruckwerte rPP als Differenzen zwischen den lokalen retinalen arteriellen Blutdruckwerten rPa und einem Ruhe-Intraokulardruckwert IOP₀ oder einem retinalen venösen Blutdruckwert außerhalb des Augapfels RVP (für RVP > IOP₀) berechnet werden und im Druckmappingbild dargestellt werden.

Die Erfindung soll nachfolgend an Ausführungsbeispielen mit Hilfe von Zeichnungen näher beschrieben werden. Hierzu zeigen:
- Fig. 1: ein Blockschaltbild für ein erstes Ausführungsbeispiel einer erfindungsgemäßen Vorrichtung,
- Fig. 2: ein Blockschaltbild für ein zweites Ausführungsbeispiel einer erfindungsgemäßen Vorrichtung,
- Fig. 3: ein Blockschaltbild für ein drittes Ausführungsbeispiel einer erfindungsgemäßen Vorrichtung,
- Fig. 4: eine Prinzipskizze für eine Einheit zur Erzeugung und Applikation eines veränderbaren Stimulationsdruckes,
- Fig. 5: ein Diagramm, aus dem die Änderung des Intraokulardruckes IOP in Abhängigkeit von der zeitlichen Änderung des veränderbaren Stimulationsdruckes SD zu entnehmen ist und
- Fig. 6a-6c: Beispiele für Druckmappingbilder.

Das erste Ausführungsbeispiel beschreibt eine einfache Ausführung eines erfindungsgemäßen Verfahrens und eine dazu geeignete Vorrichtung, mit dem/der bevorzugt die globalen retinalen Blutdruckwerte rP manuell mit visuellen Messkriterien ohne die beschriebenen Fehlerquellen des Standes der Technik bestimmt werden können. Aus den globalen retinalen Blutdruckwerten rP kann dann, wie bereits einleitend erläutert, der globale retinale Perfusionsdruckwert rPP berechnet werden.

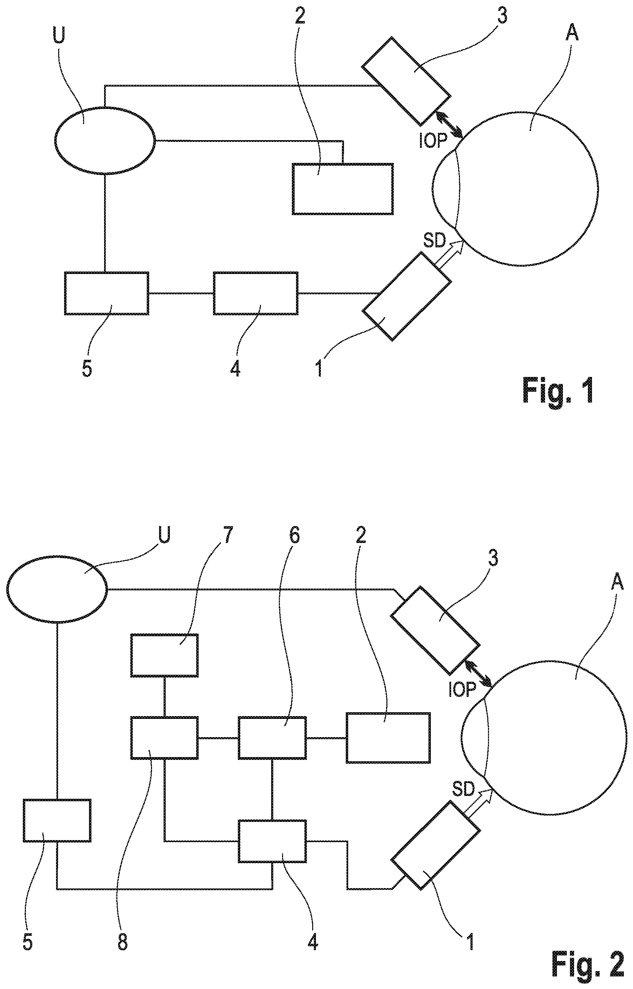

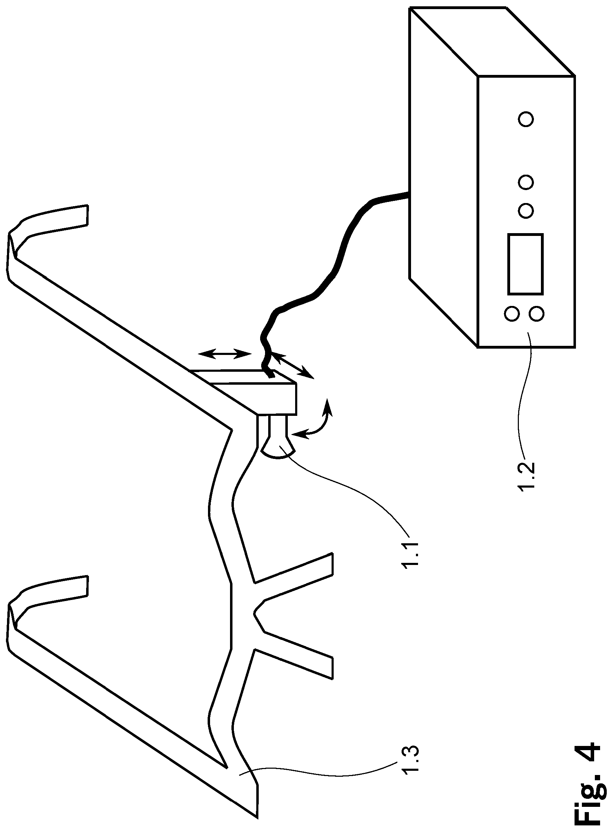

Alle Ausführungen einer erfindungsgemäßen Vorrichtung enthalten gleich diesem ersten Ausführungsbeispiel, wie in Fig. 1 in einem Blockschaltbild dargestellt, mindestens eine Einheit zur Erzeugung und Applikation eines veränderbaren Stimulationsdruckes 1, eine Rechen- und Steuereinheit 4, eine Bildgebungseinheit 2 und ein Tonometer 3. Die Einheit zur Erzeugung und Applikation eines veränderbaren Stimulationsdruckes 1, schematisch in **Fig. 4** gezeigt, weist eine Druckerzeugungseinheit 1.2, eine Halterung 1.3 und einen Druckapplikator 1.1 auf, der über die vorzugsweise brillenähnliche Halterung 1.3 jeweils seitlich (temporal) am rechten oder linken Auge A des Patienten fixiert ist. Der Druckapplikator 1.1 kann druckfrei am zu untersuchenden Auge A des Patienten flächig angelegt werden.

Der Druckapplikator 1.1 dient dem Einbringen eines veränderbaren Stimulationsdruckes SD auf das zu untersuchende Auge A des Patienten und ist gemäß dem ersten Ausführungsbeispiel ein kleiner pneumatischer Ballon, könnte aber z. B. auch ein Stempel, ein Saugnapf oder ein hydraulisches System sein.

Die Ausführung des Druckapplikators 1.1 als kleiner pneumatischer Ballon birgt im Gegensatz zu den bereits bekannten Verfahren eine Reihe von Vorteilen. So ist zum Beispiel das Verletzungsrisiko durch scharfe Kanten, welche an den Rändern von Druckapplikatoren 1.1 aus Metallen, Kunststoffen, Keramiken oder anderen festen Materialien hergestellt werden, deutlich geringer. Darüber hinaus ist die weiche Oberfläche des Ballons während der Untersuchung deutlich angenehmer für den Patienten. Zusätzlich werden durch die in alle Richtungen gleichmäßig ausgeführte Ausbreitung des Ballons Querkräfte vermieden, welche zur Verfälschung der Messergebnisse führen können.

Mit dem Druckapplikator 1.1 steht die Druckerzeugungseinheit 1.2 in Verbindung, mit der der veränderbare Stimulationsdruck SD erzeugt, erhöht, abgesenkt und konstant gehalten werden kann.

Zur gesteuerten Erzeugung des veränderbaren Stimulationsdruckes SD ist die Einheit zur Erzeugung und Applikation eines veränderbaren Stimulationsdruckes 1 mit der Rechen- und Steuereinheit 4 verbunden. Je nach gewähltem Druckapplikator 1.1 kann die Einheit zur Erzeugung und Applikation eines veränderbaren Stimulationsdruckes 1 als Druckerzeugungseinheit 1.2 z. B. eine Pumpe, ein System aus einem Pneumatikzylinder und einem Kolben oder / und eine Steuerelektronik für Linearantriebe enthalten. In diesem ersten Ausführungsbeispiel ist die Druckerzeugungseinheit 1.2 ein pneumatisches System aus einem Pneumatikzylinder und einem Kolben, welcher mit Hilfe eines Linearantriebes in dem Pneumatikzylinder verschoben werden kann. Durch die Verschiebung des Kolbens wird die im pneumatischen System enthaltene Luft komprimiert oder dilatiert, wodurch es zu einem Druckanstieg oder Druckabfall im Druckapplikator 1.1 kommt. Die Druckerzeugungseinheit 1.2 kann vorteilhaft Komponenten zur definierten Einstellung des Anstieges oder Abfalls des veränderbaren Stimulationsdruckes SD enthalten. Mögliche Ausführungen sind hierfür z. B. Systeme aus verschiedenen Drossel- und Magnetventilen oder eine geeignete Steuerelektronik, welche unterschiedliche Geschwindigkeiten bei der Verstellung eines Linearantriebs ermöglicht.

Die Druckerzeugungseinheit 1.2 weist vorteilhaft Komponenten zur Messung des veränderbaren Stimulationsdruckes SD auf. Hierfür können je nach Ausführung z. B. Drucksensoren, Kraftsensoren oder Abstandssensoren eingesetzt werden.

Die Druckerzeugungseinheit 1.2 weist vorteilhaft auch eine Komponente auf, welche ein schlagartiges Absenken des veränderbaren Stimulationsdruckes SD ermöglicht. Hierfür können zum Beispiel ein oder mehrere Magnetventile verwendet werden, mit denen das System im Notfall schlagartig entlüftet wird.

Die Halterung 1.3 dient einer direkten Kopplung des Druckapplikators 1.1 am Kopf des Patienten und kann z. B. eine Brille, ein Stirnband oder ein Bügel sein, welche über den Kopf des Patienten gelegt werden. Vorzugsweise wird die Halterung 1.3 in Form einer Brille umgesetzt. Zur Verbesserung der Kopplung des Druckapplikators 1.1 am Kopf des Patienten ist vorteilhaft an der Halterung 1.3 eine weitere Komponente, wie ein Brillenband, ein Gummiband oder eine mechanisch verstellbare Fixiermöglichkeit, vorgesehen.

Zur Realisierung einer individuell anpassbaren Positionierung des Druckapplikators 1.1, insbesondere einer Verstellbarkeit der Richtung, aus der der Druckapplikator 1.1 an das Auge A des Patienten herangeführt wird, ist der Druckapplikator 1.1 am Auge A des Patienten an der Halterung 1.3 bevorzugt über eine Höhenverstellung, eine Entfernungsverstellung und eine Winkelverstellung individuell verstellbar angebracht. Der optische Zugang zur Netzhaut für die Bildgebungseinheit 2, auch Lichtweg der Bildgebungseinheit 2, darf durch keine der in der Einheit zur Erzeugung und Applikation eines veränderbaren Stimulationsdruckes 1 enthaltenen Komponenten beeinträchtigt und / oder versperrt werden.

Als Bildgebungseinheit 2 wird im vorliegenden Beispiel eine Spaltlampe mit Hruby-Linse eingesetzt, über die der Untersucher U sich den Sehnervenkopf an der Netzhaut einstellt und beobachtet.

Das Tonometer 3 ist als Applanationstonometer ausgeführt und in bekannter Weise an die Spaltlampe montiert. Das Applanationstonometer kann mit einfachen Handgriffen gegen die Hruby-Linse ausgetauscht werden, um die Messungen des Intraokulardruckes IOP (Tonometermessungen) entsprechend dem erfindungsgemäßen Verfahren durchführen zu können.

Die Rechen- und Steuereinheit 4 ist mit der Einheit zur Erzeugung und Applikation eines veränderbaren Stimulationsdruckes 1 und einer einfachen Ein- und Ausgabeeinheit 5 signaltechnisch verbunden, womit der Untersucher U über die Ein- und Ausgabeeinheit 5 und die Rechen- und Steuereinheit 4 die Einheit zur Erzeugung und Applikation eines veränderbaren Stimulationsdruckes 1 steuern kann. Die Ein- und Ausgabeeinheit 5 ist zur Signalgebung durch den Untersucher U mit einem Doppel-Fußschalter ausgestattet, der mit der Betätigung der rechten Fußtaste den veränderbaren Stimulationsdruck SD erhöht und mit der linken Fußtaste den veränderbaren Stimulationsdruck SD verringert. Der Anstieg oder Abfall des veränderbaren Stimulationsdruckes SD wird umso schneller, je stärker die Fußtasten gedrückt werden. Wird der Fußtaster losgelassen, wird ein Stoppsignal für die Änderung des veränderbaren Stimulationsdruckes SD über die Ein- und Ausgabeeinheit 5 und die Rechen- und Steuereinheit 4 an die Einheit zur Erzeugung und Applikation eines veränderbaren Stimulationsdruckes 1 ausgelöst.

Eine Vorrichtung gemäß dem ersten Ausführungsbeispiel ist insbesondere zur Bestimmung globaler retinaler Blutdruckwerte rP vorgesehen, aus denen, wie bereits einleitend erläutert, der globale retinale Perfusionsdruckwert rPP berechnet werden kann. Sie kann aber auch durch eine mehrfache Benutzung zur Bestimmung lokaler retinaler Blutdruckwerte rP in mehreren lokal unterschiedlichen peripheren Netzhautbereichen verwendet werden.

Das Verfahren wird nachfolgend unter Verwendung einer Vorrichtung gemäß dem ersten Ausführungsbeispiel in einzelnen Verfahrensschritten beschrieben.

Schritt 1-0:
Der Untersucher U überzeugt sich davon, dass kein spontaner Venenkollaps auf dem Sehnervenkopf zu sehen ist. Ist dies der Fall, muss anstelle des Ruhe-Intraokulardruckwerts IOP₀ der retinale venöse Blutdruckwert außerhalb des Augapfels RVP zur Berechnung des retinalen Perfusionsdruckwertes rPP herangezogen werden. Vor der Untersuchung prüft der Untersucher U, dass der Druckapplikator 1.1 vollständig entlüftet ist und direkt am Auge A anliegt.

Schritt 1-1:
Der Untersucher U misst zunächst vor Beginn der Untersuchung den Ruhe-Intraokulardruck IOP₀ mit einem als Tonometer 3 verwendeten Applanationstonometer und gibt diesen zum Speichern in die Rechen- und Steuereinheit 4 ein oder protokolliert diesen.

Schritt 1-2:
Der Untersucher U tauscht das Applanationstonometer gegen die Hruby-Linse aus, stellt sich mit der Spaltlampe den Sehnervenkopf ein und startet mit der rechten Fußtaste über die Ein- und Ausgabeeinheit 5 und die Rechen- und Steuereinheit 4 das Ansteigen des veränderbaren Stimulationsdruckes SD unter Beobachtung der Venen des Sehnervenkopfes. Die Rechen- und Steuereinheit 4 steuert abhängig von der Stärke der rechten Fußtastenbetätigung die Schnelligkeit des Stimulationsdruckanstieges und löst zeitgleich mit dem ersten Betätigen das Startsignal aus. Beginnend mit dem Startsignal übernimmt die Rechen- und Steuereinheit 4 die Stimulationsdruckwerte SD von der Einheit zur Erzeugung und Applikation eines veränderbaren Stimulationsdruckes 1 und zeichnet diese in Abhängigkeit von der Zeit auf. Der Untersucher U kann zur besseren Erkennung von Pulsphänomenen mit dem Wechsel zwischen rechter und linker Fußtaste die veränderbaren Stimulationsdrücke SD beliebig schnell und lange hoch- und runterfahren.

Schritt 1-3:
Beim ersten sichtbaren Auftreten des spontanen Venenkollaps löst der Untersucher U über die Ein- und Ausgabeeinheit 5 und die Rechen- und Steuereinheit 4 das Stoppsignal aus, indem er mit den Füßen die Fußtaster verlässt, womit er das weitere Ansteigen oder Abfallen des veränderbaren Stimulationsdruckes SD in der Einheit zur Erzeugung und Applikation eines veränderbaren Stimulationsdruckes 1 stoppt und der veränderbare Stimulationsdruck SD konstant gehalten wird.

Schritt 1-4:
Der Untersucher U wechselt die Hruby-Linse gegen das Applanationstonometer und misst den momentanen Intraokulardruck IOP und protokolliert diesen Wert oder gibt diesen Wert über die Ein- und Ausgabeeinheit 5 in die Rechen- und Steuereinheit 4 ein, wo dieser Wert als retinaler venöser Blutdruck außerhalb des Augapfels RVP gemeinsam mit dem aufgezeichneten Zeitwert zum Zeitpunkt des Stoppsignals gespeichert wird.

Schritt 1-5:
Der Untersucher U beobachtet den Sehnervenkopf weiter und fährt nunmehr durch weiteres Betätigen der rechten Fußtaste den veränderbaren Stimulationsdruck SD schnell auf retinale suprasystolische Blutdruckwerte rP hoch. Die Rechen- und Steuereinheit 4 zeichnet weiterhin den veränderbaren Stimulationsdruck SD in Abhängigkeit von der Zeit auf. Nach Erreichen von retinalen suprasystolischen Blutdruckwerten rP, d. h. retinalen Blutdruckwerten rP oberhalb des retinalen arteriellen systolischen Blutdruckes rP_{asys} betätigt der Untersucher U den linken Fußschalter und fährt den veränderbaren Stimulationsdruck SD langsam wieder herunter.

Schritt 1-6:
Mit dem Erkennen der ersten arteriellen Pulsationen im Bereich des Sehnervenkopfes nimmt der Untersucher U seinen Fuß vom rechten Fußtaster, löst damit ein Stoppsignal aus, und der veränderbare Stimulationsdruck SD wird konstant gehalten. Der Untersucher U misst den Intraokulardruck IOP und protokolliert diesen oder gibt ihn über die Ein- und Ausgabeeinheit 5 in die Rechen- und Steuereinheit 4 ein. Der Intraokulardruckwert IOP wird als globaler retinaler arterieller systolischer Blutdruckwert rP_{asys} in der Rechen- und Steuereinheit 4 gespeichert und dem Zeitsignal s(t) zum Zeitpunkt des Stoppsignals zugeordnet.

Schritt 1-7:
Der Untersucher U schaut wiederum auf den Sehnervenkopf und setzt den weiteren Abfall des veränderbaren Stimulationsdruckes SD fort, bis die starken arteriellen Pulsationen auf dem Sehnervenkopf gerade verschwinden. Er nimmt den Fuß vom rechten Fußschalter, löst damit wiederum ein Stoppsignal aus, misst den Intraokulardruck IOP und gibt den erhaltenen Intraokulardruckwert IOP über die Ein- und Ausgabeeinheit 5 in die Rechen- und Steuereinheit 4 ein. Die Rechen- und Steuereinheit 4 speichert diesen Intraokulardruckwert IOP als retinalen arteriellen diastolischen Blutdruckwert rP_{adia} und ordnet diesen Wert der Zeitabhängigkeit zu.

Schritt 1-8:
Der Untersucher U beendet die Untersuchung, indem er den veränderbaren Stimulationsdruck SD schnell auf Null fährt, womit auch die Rechen- und Steuereinheit 4 den Untersuchungsvorgang abschließt, das Drucksystem komplett entlastet und die Zeitaufzeichnung beendet. Aus der Zeitaufzeichnung der Stimulationsdruck- und Intraokulardruckwerte SD, IOP wird eine individuelle Regressionsgerade gebildet, die den Zusammenhang zwischen Intraokulardruck IOP und veränderbarem Stimulationsdruck SD für jeden Stimulationsdruckwert SD berechnet. Dieser Zusammenhang wird für weitere Untersuchungen für die betreffende Person und das betreffende Auge A gespeichert. Aus den globalen retinalen Blutdruckwerten rP wird durch die Rechen- und Steuereinheit 4 nunmehr der globale retinale Perfusionsdruckwert rPP berechnet und mit den anderen retinalen Blutdruckwerten rP als Untersuchungsprotokoll ausgegeben.

Alternativ kann auf die Messung des Intraokulardruckes IOP, der dem retinalen arteriellen systolischen Blutdruck rP_{asys} gleichgesetzt wird, verzichtet werden. Nach dem Stoppsignal im Schritt 1-6 wird dann anstelle der Messung des Intraokulardruckes IOP nur ein Signal durch den Untersucher U ausgelöst, das im Zeitsignal s(t) von der Rechen- und Steuereinheit 4 gespeichert wird. Im Schritt 1-8 wird der Wert für den retinalen arteriellen systolischen Blutdruck rP_{asys} dann aus der Regressionsgeraden IOP = f(SD) berechnet. Damit kann die Zeit für die Untersuchung reduziert werden, da der retinale arterielle systolische Blutdruck rP_{asys} nicht gemessen, sondern mittels der Regressionsgeraden berechnet werden kann.

Um die Berechnung des Zusammenhangs zwischen dem Intraokulardruck IOP und dem veränderbaren Stimulationsdruck SD exakter zu gestalten, können während des beschriebenen Verfahrens weiter Stopps mit IOP-Messungen eingefügt werden.

Um Fehler durch den Tonografieeffekt zu vermeiden, werden die Tonometermessungen innerhalb von maximal 1 min abgeschlossen. Beim Einsatz eines Rebound-Tonometers können die IOP-Messungen in wenigen Sekunden durchgeführt werden.

Das vorgeschlagene erfindungsgemäße Verfahren kann sinngemäß auch verwendet werden, um nur einen der retinalen Blutdrücke rP, wie z. B. den retinalen venösen Blutdruck außerhalb des Augapfels RVP, zu messen. In diesem Fall wird der Untersuchungsvorgang bereits nach dem Schritt 1-4 abgebrochen und es kann auch auf die Messung des veränderbaren Stimulationsdruckes SD mit der Einheit zur Erzeugung und Applikation eines veränderbaren Stimulationsdruckes 1 verzichtet werden, da eine individuelle Beziehung zwischen dem Intraokulardruck IOP und dem veränderbaren Stimulationsdruck SD nicht erforderlich ist.

Mit einem zweiten Ausführungsbeispiel für eine erfindungsgemäße Vorrichtung und einem zweiten Ausführungsbeispiel für ein erfindungsgemäßes Verfahren werden vom Untersucher U gleich dem ersten Ausführungsbeispiel manuell nach visuellen Messkriterien globale retinale Blutdruckwerte rP online und anschließend zusätzlich lokale retinale Blutdruckwerte rP durch eine Offline-Auswertung einer von der ersten Untersuchung aufgezeichneten Videosequenz bestimmt.

Aus den lokalen retinalen arteriellen Blutdruckwerten rPa lassen sich auch näherungsweise lokale retinale Perfusionsdruckwerte rPP bestimmen. Da die venöse Strombahn bis zum Austritt der venösen Gefäße aus dem Augapfel gewöhnlich das Niedrigdruckgebiet darstellt und auch nur sehr moderate venöse Strömungswiderstände erwarten lässt, wird näherungsweise davon ausgegangen, dass der lokale retinale Perfusionsdruckwert rPP sich aus dem mit dem Verfahren ermittelten lokalen retinalen arteriellen Blutdruckwert rPa abzüglich des Ruhe-Intraokulardruckwertes IOP₀ oder retinalen venösen Blutdruckwertes außerhalb des Augapfels RVP (je nach dem größeren Wert) berechnet.

Die manuelle Bestimmung lokaler retinaler Blutdruckwerte rP bedeutet, dass man wiederum visuelle Pulskriterien, wie sie für die Bestimmung der globalen retinalen Blutdruckwerte rP am Sehnervenkopf beobachtet wurden, in ausgewählten Gefäßbereichen an Gefäßabschnitten und / oder ausgewählten Kapillarbereichen beobachtet, um die zugehörigen retinalen Blutdruckwerte rP an den beobachteten Stellen von der Netzhaut zu ermitteln und ggf. zugehörige retinale Perfusionsdruckwerte rPP zu errechnen.

Lokale visuelle Pulskriterien sind vor allem die schnelle Zunahme von heftigen Durchmesserpulsationen bis hin zu Gefäßverschlüssen oder auch kapillare pulsähnliche Abblassungen bis zum kompletten Kapillarverschluss, der als komplette Abblassung erkennbar wird. Erfindungsgemäß werden aus der Mikrozirkulation definierte lokale retinale Blutdrücke rP, wie z. B. der kritische retinale arterielle oder kapillare Blutdruck (retinaler arterieller kritischer Blutdruck rPaₖᵣᵢₜ) oder der retinale arterielle oder kapillare Verschlussdruck (retinaler arterieller Verschlussdruck rPa_{VS}), benutzt, die bei Reduzierung des retinalen Perfusionsdruckes rPP mit heftigen Pulsationen bei einem retinalen arteriellen kritischen Blutdruckwert rPaₖᵣᵢₜ bis zum Gefäßverschluss, der bei einem retinalen arteriellen Verschlussdruckwert rPa_{VS} auftreten und als spezielle pathophysiologische, definierte lokale retinale Blutdrücke rP bestimmt werden.

Die Bestimmung lokaler retinaler Blutdruckwerte rP, insbesondere der retinale arterielle Verschlussdruckwert rPa_{VS}, ist besonders von klinischem Vorteil bei lokalen Durchblutungsstörungen der Netzhaut, wie z. B. bei Astverschlüssen oder wenn lokale Gefäßbereiche ausgefallen oder eingeschränkt sind, wie z. B. im Falle der diabetischen Retinopathie, beim Glaukom und anderen Erkrankungen. Retinale arterielle kritische Blutdrücke rPaₖᵣᵢₜ und retinale Perfusionsdrücke rPP haben andererseits prädiktiven Wert und können eine funktionelle Früherkennung kritischer Durchblutungszustände ermöglichen.

Das zweite Ausführungsbeispiel stellt sowohl aus Sicht der Vorrichtung als auch des Verfahrens eine erfindungsgemäße Erweiterung des ersten Ausführungsbeispieles dar.

Die erfindungsgemäße Erweiterung der Vorrichtung ist in **Fig. 2** dargestellt. Anstelle der Spaltlampe gemäß dem ersten Ausführungsbeispiel wird nunmehr als Bildgebungseinheit 2 eine Netzhautkamera mit digitaler Bildaufnahmeeinheit verwendet, die mit einem digitalen Videorekorder 6 verbunden ist, dem die aufgenommene Videosequenz zugeleitet wird. Darüber hinaus enthält die Vorrichtung eine Ergebnisspeichereinheit 7 und eine damit verbundene Daten- und Bildauswerteeinheit 8, die wiederum, ebenso wie der digitale Videorekorder 6, mit der zentralen Rechen- und Steuereinheit 4 über Signalwege verbunden ist. Die Ein- und Ausgabeeinheit 5 enthält einen Monitor zur Darstellung der Videosequenzen und zur Ergebnispräsentation. Vorteilhaft wird als Tonometer 3 kein Applanationstonometer eingesetzt, sondern ein Rebound- Tonometer.

Der digitale Videorekorder 6 ist eingangsseitig über einen Signalweg mit der Rechen- und Steuereinheit 4 verbunden, um auf ein Zeitsignal s(t) synchron mit der Einheit zur Erzeugung und Applikation eines veränderbaren Stimulationsdruckes 1 angesteuert zu werden. Über diese Signalverbindung zur Rechen- und Steuereinheit 4 werden auch die Steuersignale, durch den Untersucher U mit Hilfe der Ein- und Ausgabeeinheit 5 ausgelöst bzw. in die Rechen- und Steuereinheit 4 eingegeben, an die Bildgebungseinheit 2 weitergeleitet. Der beschriebene Signalweg dient somit der Ablaufsteuerung der Vorrichtung. Die Rechen- und Steuereinheit 4 ordnet dann zeitlich diesem Zeitsignal s(t) alle ermittelten Intraokulardruckwerte IOP bzw. globalen retinalen Blutdruckwerte rP zu, aber auch die später bei der Offline-Auswertung anfallenden lokalen retinalen Blutdruckwerte rP mit den den lokalen retinalen Blutdruckwerten rP zugeordneten Bildern und Messorten im Bild.

Der digitale Videorekorder 6 ist über einen weiteren Signalweg mit der Daten- und Bildauswerteeinheit 8 verbunden, wo eine Verarbeitung und synchronisierte Speicherung aller Daten und Steuersignale auf das Zeitsignal s(t) erfolgt. Die Daten- und Bildauswerteeinheit 8 wird ebenfalls über die Rechen- und Steuereinheit 4 angesteuert, mit der sie über einen Signalweg verbunden ist.

Mit Hilfe der Signalwege wird über die Rechen- und Steuereinheit 4 eine Ablaufsteuerung des nachfolgend noch näher beschriebenen Verfahrens realisiert. Die durch den Untersucher U über die Ein- und Ausgabeeinheit 5 eingegebenen Steuerbefehle werden über eine Signalleitung an die Rechen- und Steuereinheit 4 weitergeleitet, dort verarbeitet und an die betreffenden Einheiten weitergeleitet.

Die Ein- und Ausgabeeinheit 5 dient zur Eingabe von Daten und Steuerbefehlen durch den Untersucher U und zur Darstellung und Ausgabe der jeweiligen Untersuchungsergebnisse. Während der Untersuchung werden die Stimulationsdruckwerte SD und die Videosequenz auf dem Monitor online dargestellt. Der Untersucher U beobachtet in diesem zweiten Ausführungsbeispiel den Sehnervenkopf über den Monitor und nicht mehr direkt über die Spaltlampe wie im ersten Ausführungsbeispiel.

Erfindungsgemäß wird nach der Bestimmung der globalen retinalen Blutdruckwerte rP, die entsprechend der im ersten Ausführungsbeispiel erfolgten Verfahrensschritte durchgeführt wird, die Offline-Auswertung lokaler retinaler Blutdruckwerte rP anhand der aufgezeichneten Videosequenz durchgeführt. Die beiden Fußschalter (rechts und links) werden dann zur Steuerung der Videowiedergabe betätigt (die Geschwindigkeit der Videowiedergabe wird über die Stärke der Betätigung der Fußschalter gesteuert, linker Fußschalter fährt die Videosequenz zurück, rechter Fußschalter steuert die Wiedergabe vorwärts). Eine Freigabe des Fußschalters stoppt die Wiedergabe der Videosequenz und es wird ein Bild der Videosequenz als Standbild auf dem Monitor präsentiert. Auf dem Monitor wird der aktuelle, zum präsentierten Bild gehörige Stimulationsdruckwert SD und / oder wahlweise der zugeordnete Intraokulardruckwert IOP dargestellt. Grundlage dafür ist die bereits nach Abschluss der Bestimmung der globalen retinalen Blutdruckwerte rP vorliegende Berechnung des Zusammenhanges aus dem veränderbaren Stimulationsdruck SD und dem Intraokulardruck IOP.

Die Verfahrensschritte zur Offline-Messung lokaler retinaler Blutdrücke rP laufen im Anschluss an die Verfahrensschritte zur Bestimmung der globalen retinalen Blutdruckwerte rP wie folgt ab:
Schritt 2-1:
   Der Untersucher U startet die Offline-Auswertung über die Ein- und Ausgabeeinheit 5 (Ablaufmenüpunkt). Die Ergebnisbildspeichereinheit 7 und das Zeitsignal s(t) werden auf s(t) = Null gesetzt. Der digitale Videorekorder 6 fährt das erste Bild an. Eine Grafik stellt im Bild den ersten Stimulationsdruckwert SD bzw. Intraokulardruckwert IOP auf dem Monitor dar. Die Ein- und Ausgabeeinheit 5 wird auf interaktiven Modus im Monitorbild geschaltet und es erscheint ein Kursor im Bild (Standbild) der Netzhaut zur Markierung von Messorten mittels einer zur Ein- und Ausgabeeinheit 5 gehörigen Maus durch den Untersucher U. Die Fußschalter werden auf Videosteuerung wie oben beschrieben umgestellt.
Schritt 2-2:
   Der Untersucher U beginnt die Videosequenz im Vor- und Rücklauf per Füße zu steuern und beobachtet dabei die interessanten Gefäßbereiche. Bei Auftreten der weiter oben beschriebenen Pulsphänomene stoppt er die Wiedergabe.
Schritt 2-3:
   Der Untersucher U markiert mit der Maus im Standbild die Stelle mit dem Pulsphänomen als Messort im Bild, klickt anschließend in einer Liste den zum Pulsphänomen zugehörigen retinalen Blutdruckwert rP an. Die Rechen- und Steuereinheit 4 übernimmt den zum Bild gehörigen retinalen Blutdruckwert rP bzw. den Stimulationsdruckwert SD, rechnet den Stimulationsdruckwert SD ggf. in den retinalen Blutdruckwert rP um und speichert das Ergebnis gemeinsam mit einer Bildnummer eines der dem Zeitsignal s(t) zugeordneten Bilder des Videosignals und dem angegebenen Messort im Bild im Untersuchungsprotokoll. Der Untersucher U wird aufgefordert, einen zugehörigen venösen Messort zur Berechnung des retinalen Perfusionsdruckwertes rPP im weiteren Schritt anzugeben. Im Fall retinaler arterieller kritischer Blutdruckwerte rPaₖᵣᵢₜ kann der Untersucher U auch anstelle eines arteriellen und venösen Messortes den betreffenden (abgeblassten) Kapillarbereich mit der Maus grafisch umschreiben.
   Nach Abschluss dieses Schrittes kehrt der Untersucher U bei gewünschter Fortsetzung zur Erfassung weiterer Messorte und weiterer retinaler Blutdrücke rP zu Schritt 2-2 zurück oder beendet das Verfahren.
Schritt 2-4:
   Bei Beendigung des Verfahrens werden alle retinalen Blutdruckwerte rP mit ihren Messorten in ein Druckmappingbild eingetragen, dem Untersucher U präsentiert und als Messprotokoll zahlenmäßig ausgedruckt.

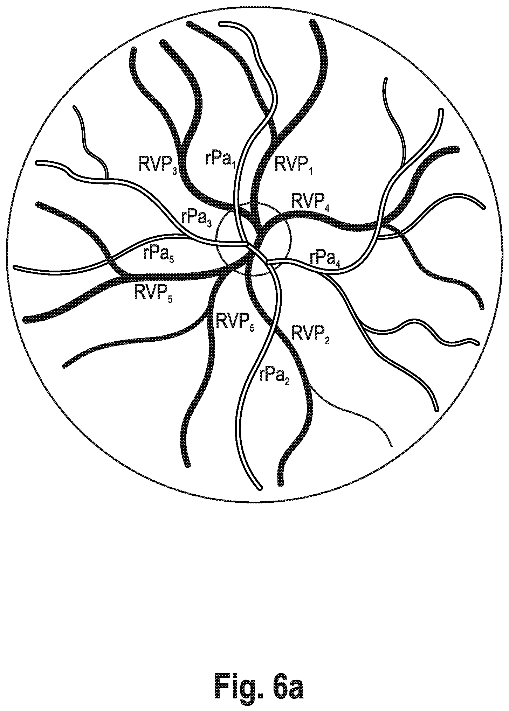

Zur Realisierung der auf die eigentliche Untersuchung folgenden Offline-Auswertung dient die Daten- und Bildauswerteeinheit 8. Diese lädt die während der Untersuchung aufgezeichneten Daten und verrechnet diese miteinander, so dass durch ein Druckmapping ein Druckmappingbild, wie in den **Fig. 6a-6c** gezeigt, erstellt werden kann, das eine visuelle Betrachtung der lokalen Perfusionsdruckunterschiede ermöglicht wird.

Mit einem dritten Ausführungsbeispiel für eine erfindungsgemäße Vorrichtung und ein erfindungsgemäßes Verfahren wird über die Bestimmung von globalen und lokalen retinalen Blutdruckwerten rP hinaus ein objektives und automatisches Abbilden der Netzhaut (Netzhautimaging) durchgeführt.

Unter stationären Bedingungen der Mikrozirkulation des Blutes baut sich ein Netzwerk von Strömungswegen mit unterschiedlichen Strömungswiderständen auf, die die Kapillargebiete selbst, aber auch die den Kapillargebieten vor- und nachgeschalteten Gefäßabschnitte, jeweils lokale Gefäßnetze bildend, betreffen. Das hat eine örtliche Verteilung von retinalen Perfusionsdrücken rPP zwischen den einzelnen lokalen Gefäßnetzen und insbesondere einzelnen lokalen Kapillarbereichen zur Folge.

Die lokalen Unterschiede des Strömungswiderstandes führen dazu, dass bei steigendem Intraokulardruck IOP oder abfallendem retinalen Perfusionsdruck rPP bzw. abfallendem retinalen arteriellen Blutdruck rPa die lokalen Gefäßnetze bzw. Kapillarbereiche mit hohen vor- oder nachgeschalteten Strömungswiderständen bereits zu einem früheren Zeitpunkt kollabieren oder nicht mehr ausreichend mit Blut versorgt werden können, während andere benachbarte Gefäßbereiche mit einem niedrigeren vor- oder nachgeschalteten Strömungswiderstand noch ausreichend durchblutet sind.

Das Kollabieren größerer Gefäße ist an stark zunehmenden Pulsationen der Gefäßdurchmesser erkennbar und als objektives Messkriterium für retinale arterielle Verschlussdrücke rPa_{VS} nutzbar. Das Erreichen lokaler retinaler arterieller kritischer Blutdruckwerte rPaₖᵣᵢₜ, wenn lokale retinale arterielle diastolische Blutdruckwerte rP_{adia} überschritten werden, wird in Kapillarbereichen ebenfalls zuerst durch ein Ansteigen der Pulsationen der betroffenen Bereiche gekennzeichnet, worauf bei weiter steigendem Intraokulardruck IOP ein Verschluss der Kapillaren und ein daraus resultierendes Abblassen und / oder Ergrauen des jeweiligen Kapillarbereiches folgt. Zum Zeitpunkt des kompletten Verschlusses eines Kapillargebietes erreicht dieses Kapillargebiet im Bild der Netzhaut seinen maximalen Helligkeitswert. Das Erreichen eines örtlichen Maximums der Helligkeit kann als objektives Messkriterium für das Erreichen eines retinalen arteriellen Verschlussdruckes rPa_{VS} und der Beginn der starken kapillaren Pulsationen (bzw. der Helligkeit des Kapillarbereiches) kann als objektives Messkriterium für einen retinalen arteriellen kritischen Blutdruck rPaₖᵣᵢₜ verwendet werden. Wesentlich für die erfindungsgemäße Ausgestaltung ist, dass im Unterschied zur üblichen Messung retinaler arterieller Blutdrücke rPa der Intraokulardruck IOP nicht von retinalen suprasystolischen Blutdruckwerten rP abfallen bzw. abgesenkt werden darf, sondern von dem Ruhe-Intraokulardruckwert IOP₀ vor der Untersuchung ansteigen bzw. angehoben werden muss, da sich ansonsten die gesuchten Druckdifferenzen nicht herausbilden können (siehe Verfahrensschritte).

Das Verfahren gemäß dem dritten Ausführungsbeispiel kann vorteilhaft in der Untersuchung der kapillaren Regelreserve sowie als Prädiktor für zu erwartende Kapillarausfälle bei diabetischer Retinopathie, Glaukom und anderen Erkrankungen angewendet werden.

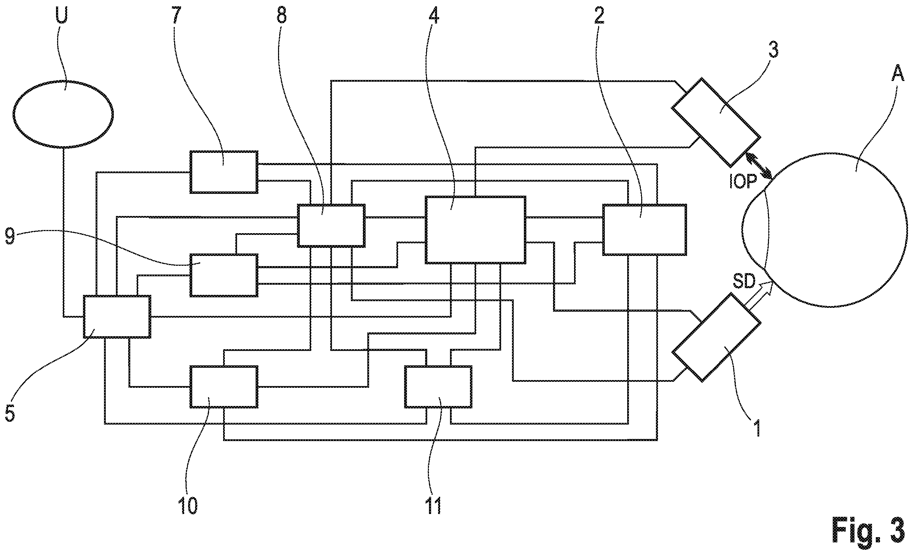

Wie in **Fig. 3** in einem Blockschaltbild dargestellt, baut auch das dritte Ausführungsbeispiel auf den vorangegangenen Beispielen auf und erweitert diese. Die Vorrichtung des zweiten Ausführungsbeispiels wird um eine Signalanalyseeinheit 9, um eine Einheit zur Bildung von spektralen Quotientensignalen 10 und um eine Einheit zur Bildung von Gefäßdurchmessersignalen 11 erweitert. Die Bildgebungseinheit 2 ist hier eine spektral modifizierte Netzhautkamera und der digitale Videorekorder 6 entfällt. Alle Einheiten 9, 10, 11 sind mit der Ein- und Ausgabeeinheit 5 und der Daten- und Bildauswerteeinheit 8 sowie mit der Bildgebungseinheit 2 verbunden. Die Videosequenz wird vorzugsweise dem Untersucher U gemeinsam mit Messergebnissen und den retinalen Blutdruckwerten rP zur Einstellung der Netzhaut und zur Verfolgung und Kontrolle der Einstellung während des Untersuchungsvorganges präsentiert.

Die Einheit zur Bildung von spektralen Quotientensignalen 10 dient der Ausschaltung der Abhängigkeit der Beleuchtungsintensität auf die für die Untersuchung gebildeten Signale. Die spektral modifizierte Netzhautkamera stellt die Bildgebungseinheit 2 dar. Sie weist im Beleuchtungsstrahlengang erfindungsgemäß einen mindestens doppelbandigen Bandpassfilter auf, z. B mit einem Spektralbereich im roten und einem Spektralbereich im grünen Licht, der jeweils auf die spektrale Empfindlichkeit eines roten und grünen Farbkanals des digitalen Bildsensors abgestimmt ist. Die Abstimmung erfolgt derart, das weder der rote, noch der grüne Farbkanal für den jeweils anderen Spektralbereich des Bandpassfilters empfindlich ist. Die Farbkanäle und der Bandpassfilter sind Bestandteile der spektral modifizierten Netzhautkamera. Die Einheit zur Bildung von spektralen Quotientensignalen 10 bekommt die Videosignale der Netzhautkamera zugeleitet und bildet pixelweise Quotienten aus den Intensitäten des roten Farbkanals dividiert durch den grünen Farbkanal, wobei die Pixel dem gleichen Netzhautort im Netzhautbild entsprechen müssen. Damit entsteht ein spektrales normiertes Quotientenbild, bei dem beleuchtungsseitige Unterschiede durch spektrale Normierung beseitigt sind. Das rote rückgestreute Licht, das Blut im Wesentlichen durchstrahlt, dient dabei als Referenzwellenlänge, wobei Licht im grünen Licht stark von Blut absorbiert wird und das Blutvolumen in einem Netzhautbereich widerspiegelt. Der Quotient beschreibt beleuchtungsunabhängig das Blutvolumen in einem Kapillarbereich. Die dadurch entstehende Quotientenbildsequenz von der Netzhaut wird in der Einheit zur Bildung von spektralen Quotientensignalen 10 gespeichert und dann entsprechend der Verfahrensschritte an die Signalanalyseeinheit 9 geleitet.

Die Einheit zur Bildung von Gefäßdurchmessersignalen 11 bestimmt Gefäßdurchmesser in selektierten Gefäßabschnitten segmentweise entlang der Gefäßabschnitte sowie von Bild zu Bild im grünen Farbkanal der Videosequenz oder wahlweise im Quotientenbild. Aus der zeitlichen Folge der Gefäßdurchmesser der einzelnen Gefäßsegmente werden dann Gefäßdurchmessersignale gebildet, die der Signalanalyseeinheit 9 zugeleitet werden.

Die Erfindung muss nicht zwangsweise beide, sowohl eine Einheit zur Bildung von spektralen Quotientensignalen 10 und eine Einheit zur Bildung von Gefäßdurchmessersignalen 11, besitzen und muss auch nicht zwangsweise spektral normierte Quotientenbilder und daraus abgeleitete Signale bilden. Die vorgestellten Vorschläge dieses Ausführungsbeispiels stellen vorteilhafte Ausführungen dar.

Das Tonometer 3 ist hier über Signalwege mit der Rechen- und Steuereinheit 4 und der Daten- und Bildauswerteeinheit 8 verbunden. Es wird im Gegensatz zu den bereits beschriebenen Ausführungsbeispielen nicht mehr manuell vom Untersucher U bedient, welcher die gemessenen retinalen Blutdrücke rP über die Ein- und Ausgabeeinheit 5 eingeben muss, sondern direkt in die Vorrichtung integriert und vollautomatisch von dieser gesteuert. Um dies zu realisieren, ist das Tonometer 3 über einen Signalweg mit der Rechen- und Steuereinheit 4 verbunden. Diese Verbindung dient der Übertragung der Intraokulardruckwerte IOP, welche beim Erreichen zuvor definierter Messkriterien eine automatisch durchgeführte Messung des Intraokulardrucks IOP auslösen. Die ermittelten Intraokulardruckwerte IOP werden über einen Signalweg an die Rechen- und Steuereinheit 4 übermittelt und dort zur weiteren Verarbeitung auf das Zeitsignal s(t) synchronisiert. Die auf das Zeitsignal s(t) synchronisierten Intraokulardruckwerte IOP werden zur Speicherung und weiteren Verarbeitung über einen Signalweg an die Daten- und Bildauswerteeinheit 8 gesendet.

Die Ergebnisspeichereinheit 7 dient auch in diesem Ausführungsbeispiel der Speicherung bzw. Zwischenspeicherung von Druckmappingbildern.

Als Gefäßdurchmessersignale, die der Signalanalyseeinheit 9 zur Analyse übergeben werden, dienen der zeit- und ortsabhängige Gefäßdurchmesser einzelner Gefäßsegmente oder der für einen aus mehreren Gefäßsegmenten gebildeten Gefäßabschnitt gemittelte Gefäßdurchmesser aus der Einheit zur Bildung von Gefäßdurchmessersignalen 11, sowie die über eine definierte Fläche, gebildet durch ein Pixel oder eine Gruppe von Pixeln, gemittelte unnormierte Helligkeit oder / und der gemittelte Quotient aus Helligkeitswerten unterschiedlicher Farben.

Zur Ausführung der automatischen Messung der globalen retinalen Blutdrücke rP und zum Druckmapping werden folgende Verfahrensschritte durchgeführt.

Schritt 3-0:
Der Untersucher U bringt den Druckapplikator 1.1 am Kopf des Patienten derart an, dass der Druckappliator 1.1, ohne Druck auszuüben, das Auge A im temporalen Lidwinkel leicht berührt.

Dann stellt er die modifizierte Netzhautkamera und das integrierte Tonometer 3 derart auf das Auge A ein, dass parallel zur Netzhautbildgebung automatische Tonometermessungen möglich sind und die Netzhautkamera auf dem Monitor der Ein- und Ausgabeeinheit 5 ein auswertbares Bild von der Netzhaut mit dem Sehnervenkopf liefert.

Die von der Bildgebungseinheit 2 gelieferte Videosequenz wird mit geeigneten Mitteln auf ausreichende Bildqualität geprüft. Gegebenenfalls wird der Untersucher U aufgefordert, durch Einstellung der Netzhautkamera die Bildqualität zu korrigieren.

Dann startet der Untersucher U den Untersuchungsvorgang.

Schritt 3-1:
Die Rechen- und Steuereinheit 4 veranlasst eine automatische Intraokulardruckmessung als Ausgangswert bzw. Ruhe-Intraokulardruckwert IOP₀.

Schritt 3-2-1:
Der veränderbare Stimulationsdruck SD wird jetzt erhöht, während von der Netzhaut eine Videosequenz bildende Bilder der Netzhaut erzeugt werden. Die Daten- und Bildauswerteeinheit 8 analysiert die Bilder der Videosequenz und bestimmt Bildverschiebungen bzw. Verdrehungen zwischen benachbarten Bildern und korrigiert die Bildkoordinaten derart, dass eine bewegungskorrigierte Videosequenz erzeugt wird, bei der sich gleiche Netzhautpunkte überdecken. Nachfolgend wird von dieser bewegungskorrigierten Videosequenz ausgegangen.

Die Papille (Sehnervenkopf) und die Gefäße werden mit geeigneten Mitteln selektiert, wobei anhand der Farbbilder oder der Quotientenbilder arterielle und venöse Gefäße voneinander getrennt werden. Das selektierte arterielle und venöse Gefäßnetz wird gespeichert.

Schritt 3-2-2:
Die Einheit zur Bildung von Gefäßdurchmessersignalen 11 greift auf das selektierte Gefäßnetz zu und bestimmt segmentweise entlang der Gefäße sowie von Bild zu Bild Gefäßdurchmesser, deren Wert jeweils dem Ort, der Zeit bzw. dem Bild zugeordnet gespeichert wird.

Schritt 3-2-3:
Die Einheit zur Bildung von spektralen Quotientensignalen 10 bildet aus den bewegungskorrigierten Bildern der Videosequenz, wie weiter oben beschrieben, spektral normierte Quotientenbilder.

Weiterhin werden für alle Pixel der Quotientenbilder, die nicht als Gefäß erkannt wurden und damit nicht zum selektierten Gefäßnetz gehören, Quotientensignale erstellt, die den zeitlichen Verlauf des Quotientensignals pro Pixel am Messort auf der Netzhaut über das Zeitsignal s(t) in den Bildern der Videosignale beschreiben.

Schritt 3-2-4:
Die Daten- und Bildauswerteeinheit 8 bildet aus den roten oder grünen bewegungskorrigierten Bildern (Farbbildern) des Videosequenz ebenfalls den Zeitverlauf der zeit- und ortsabhängigen grünen und / oder roten Farbintensitätssignale für alle Pixel, ausgenommen der Pixel, die zum selektierten Gefäßnetz gehören.

Alle gebildeten Signale werden der Signalanalyseeinheit 9 zugeleitet.

Schritt 3-3:
Die Signalanalyseeinheit 9 überwacht alle Signale bezüglich der nachfolgend definierten objektiven Messkriterien.

Schritt 3-3-1:
Die Signale auf dem selektierten Sehnervenkopf werden bezüglich des Auftretens eines spontanen Venenkollaps überwacht. Als objektives Messkriterium werden benutzt:
a) Einzelne venöse Gefäßsegmente beginnen stärker um ein Vielfaches zu pulsieren als zuvor und / oder zugleich stärker als die meisten venösen Gefäßsegmente auf dem Sehnervenkopf. Der Schwellenwertfaktor für die sich daraus ergebene Änderung des Durchmessers wird mit Faktor 3 festgelegt, kann aber anhand experimenteller Untersuchungen anders eingestellt werden.
b) Die Quotientensignale und / oder die roten Farbintensitätssignale und / oder die grünen Farbintensitätssignale steigen in ihrer Pulsamplitude um ein Vielfaches gegenüber zuvor und / oder gegenüber den benachbarten Pixeln an. Als Schwellenwertfaktor wird der Faktor 3 festgelegt, der aber anhand experimenteller Untersuchungen anders oder zwischen den verschiedenen Signalen auch unterschiedlich eingestellt werden kann.

Schritt 3-3-2:
Die genannten Signale der gesamten Netzhaut werden nach folgenden lokalen retinalen arteriellen Blutdrücken rPa überwacht:
rP_{adia}: retinaler arterieller diastolischer Blutdruck:
   Kriterium: Segmente des arteriellen Gefäßdurchmessers beginnen in ihrer zeitlichen Pulsamplitude um mindestens das 3-fache der Pulsamplitude bei kleineren Stimulationsdruckwerten SD anzusteigen
rPaₖᵣᵢₜ: retinaler arterieller kritischer Blutdruck
   Kriterium: beginnende zunehmende Pulsationen der Quotientensignale bzw. der roten Farbintensitätssignale und der grünen Farbintensitätssignale um das 3-fache der bei kleineren Stimulationsdruckwerten SD beobachtbaren retinalen Blutdruckwerte rP
rPa_{VS}: retinaler arterieller Verschlussdruck
   Kriterium: starke Pulsationen der Quotientensignale bzw. der roten Farbintensitätssignale und der grünen Farbintensitätssignale sind nicht mehr nachweisbar und die Quotientensignale bzw. die roten Farbintensitätssignale und die grünen Farbintensitätssignale sind deutlich gegenüber den Werten vor den starken Pulsationen, um mindestens die Hälfte der Pulsamplitude angestiegen
rP_{asys}: retinaler arterieller systolischer Blutdruck
   Kriterium: die starken arteriellen Durchmesserpulsationen sind auf mindestens ein Drittel der Hälfte der diastolischen Pulsamplitude zusammengebrochen

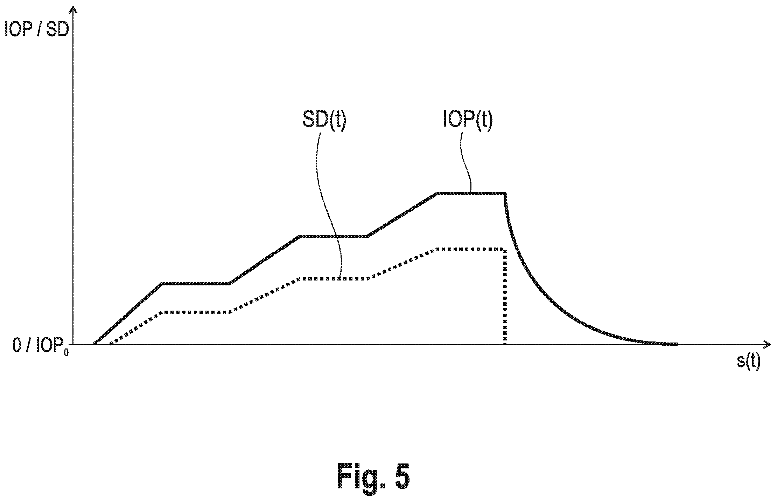

Schritt 3-4:
Erkennt die Signalanalyseeinheit 9, dass mindestens eines der Kriterien für den Venenkollaps erfüllt ist, wird der Ruhe-Intraokulardruckwert IOP₀ dem retinalen venösen Blutdruckwert rPv gleichgesetzt. In diesem Fall bestimmt der Ruhe-Intraokulardruckwert IOP₀ den retinalen Perfusionsdruckwert rPP venöserseits. In diesem Fall, aber auch wenn der spontane Venenkollaps nicht erkannt wurde, wird über die Rechen- und Steuereinheit 4 der Start der Einheit zur Erzeugung und Applikation eines veränderbaren Stimulationsdruckes 1 und das Ansteigen des veränderbaren Stimulationsdruckes SD ausgelöst. Der veränderbare Stimulationsdruck SD soll mit mindestens 1 mmHg pro Sekunde ansteigen. Für den nachfolgenden zeitlichen Ablauf siehe **Fig. 5****.**

Die Signalanalyseeinheit 9 überwacht weiterhin den Eintritt von Messkriterien. Die Rechen- und Steuereinheit 4 ordnet alle aktuellen Stimulationsdruckwerte SD einem Zeitsignal s(t) zu, das mit dem ersten Startsignal gleich Null gesetzt wird und dem ab diesem Zeitpunkt auch alle originalen und abgeleiteten Bilder der Videosequenz, Quotientenbilder und Signale zugeordnet werden.

Schritt 3-5:
Erkennt die Signalanalyseeinheit 9 den spontanen Venenkollaps auf dem Sehnervenkopf, dann wird über die Rechen- und Steuereinheit 4 das Stoppsignal für die Einheit zur Erzeugung und Applikation eines veränderbaren Stimulationsdruckes 1 ausgelöst und der veränderbare Stimulationsdruck SD wird konstant gehalten. Die Rechen- und Steuereinheit 4 löst eine automatische Tonometermessung aus. Der zum Stoppzeitpunkt gemessene Intraokulardruckwert IOP wird dem Zeitsignal s(t) zugeordnet. Nach Speicherung und Zuordnung des Intraokulardruckwertes IOP als Wert für den retinalen venösen Blutdruck außerhalb des Augapfels RVP erfolgt die Fortsetzung des Stimulationsdruckanstieges.

Schritt 3-6:
Tritt ein weiteres Messkriterium ein, wird über die Rechen- und Steuereinheit 4 wiederum das Stoppsignal für die Einheit zur Erzeugung und Applikation eines veränderbaren Stimulationsdruckes 1 ausgelöst, der veränderbare Stimulationsdruck SD wird nicht weiter erhöht und der zugehörige Intraokulardruckwert IOP wird durch Auslösen einer automatischen Tonometermessung durch die Rechen- und Steuereinheit 4 bestimmt. Der Intraokulardruckwert IOP wird wiederum dem Zeitsignal s(t), aber auch dem Messort bzw. den Messorten im Netzhautbild und dem zum Messkriterium gehörenden retinalen Blutdruckwert rP zugeordnet.

Wurden mindestens zwei Tonometermessungen unter verschiedenen erhöhten veränderbaren Stimulationsdrücken SD durchgeführt, wird der individuelle Zusammenhang zwischen den Intraokulardruckwerten IOP im Auge A und den Stimulationsdruckwerten SD (IOP=f(SD)) bestimmt. Weitere Intraokulardruckwerte IOP können zur Erhöhung der Genauigkeit dieses Zusammenhanges bestimmt werden. Der Zusammenhang IOP=f(SD) wird gespeichert und kann zur Berechnung beliebiger Intraokulardruckwerte IOP aus den aktuellen Stimulationsdruckwerten SD für die vorliegende Untersuchung benutzt werden.

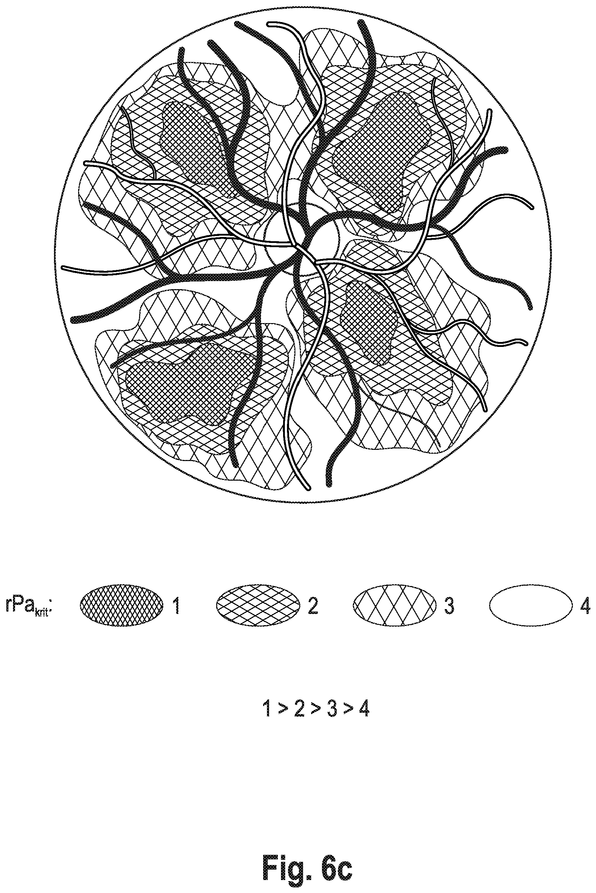

Schritt 3-7:
Treten Messkriterien für retinale arterielle kritische Blutdrücke rPaₖᵣᵢₜ oder retinale arterielle Verschlussdrücke rPa_{VS} auf, werden die zusammenhängenden Messorte auf der Netzhaut zu Messbereichen B bzw. Flächenelementen zusammengefasst und es werden Grenzlinien dieser Messbereiche B gebildet. Zu jedem Bild mit diesen Messbereichen B kann ein retinaler Blutdruckwert rP zugeordnet werden und eine dynamische Darstellung der kritischen oder verschlossenen Messbereiche B präsentiert werden. Die Entwicklung dieser Messbereiche B kann auch in einem Ergebnisbild farblich kodiert zusammengefasst werden. Ein solches Ergebnisbild stellt ein Druckmappingbild dar und ist in **Fig. 6c** gezeigt. Die unterschiedlich schraffierten Messbereiche B, in denen jeweils mehrere Messorte liegen, haben unterschiedlich hohe Werte, z. B. für den retinalen Perfusionsdruck rPP, den retinalen arteriellen kritischen Blutdruck rPaₖᵣᵢₜ oder den retinalen arteriellen Verschlussdruck rPa_{VS}.

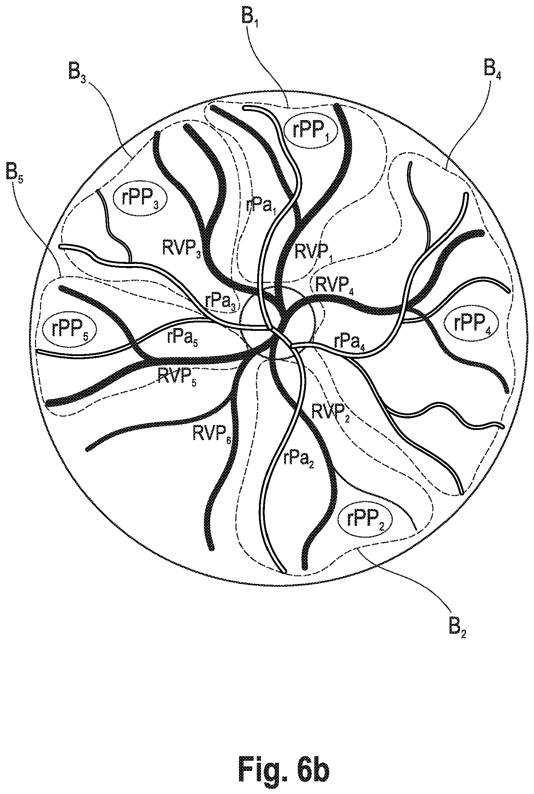

Lokale retinale Blutdrücke rP, gemessen an Gefäßabschnitten, können ebenfalls in einem Ergebnisbild als Perfusionsdruck- und Blutdruckmappingbild (Druckmappingbild) dargestellt werden, wie in den **Fig. 6a****und** **6b** gezeigt.

Schritt 3-8:
Spätestens nach dem Erreichen retinaler suprasystolischer Blutdruckwerte rP wird nach Bestimmung des zugehörigen retinalen arteriellen Blutdruckes rPa die Untersuchung beendet, und die Rechen- und Steuereinheit 4 veranlasst die schnelle Senkung des veränderbaren Stimulationsdruckes SD auf den Wert 0. Das Messprotokoll und ein Druckmappingbild, enthaltend die erfassten retinalen Blutdruckwerte rP, und ein Druckmappingbild, enthaltend die errechneten retinalen Perfusionsdruckwerte rPP, werden erstellt und ausgegeben. Die retinalen Blutdruckwerte rP und die retinalen Perfusionsdruckwerte rPP können auch in einem Druckmappingbild dargestellt werden.

Schritt 3-9:
Aus den lokalen retinalen arteriellen Blutdruckwerten rPa werden dann die lokalen retinalen Perfusionsdruckwerte rPP näherungsweise berechnet aus: $rPP . . = rPa . . - {IOP}_{0} \left({IOP}_{0}>RVP\right) bzw .$ $rPP . . = rPa . . - RVP für RVP > {IOP}_{0}$ und in das Druckmappingbild zum Perfusionsdruckmapping direkt oder zur besseren Erkennung mit unterschiedlichen Farben kodiert, z. B. rot für retinale arterielle Verschlussdrücke rPa_{VS}, gelb für retinale kritische Blut- und Perfusionsdrücke und grün für Normalwerte von retinalen Blutdruck- oder Perfusionsdruckwerten, eingetragen.

Eine vorteilhafte Ausführung kann auch der Einsatz eines bildgebenden Verfahrens auf Basis der Laser-Scanning-Technologie sein, mit dem normale Bilder vom Augenhintergrund aufgenommen werden oder über verschiedene farbliche Laser analog dem beschriebenen Verfahren auf Basis konventioneller Netzhautkameras Ausführungsvarianten der Erfindung realisiert werden.

Weitere Ausführungsvarianten ergeben sich, wenn die bildgebende Einheit als ein OCT-Gerät ausgeführt wird, d.h. die Bildgebung erfolgt auf der Basis der optischen Kohärenztomografie. Aus den aufgezeichneten OCT-Bildern werden Gefäßsignale dreidimensional gebildet und bewertet sowie Signale abgeleitet, die die lokale Blutgeschwindigkeit, den lokalen Blutfluss oder lokalen Hämatokrit (Blutzellendichte) in den großen Netzhautgefäßen oder Kapillaren beschreiben. Als Beispiel dafür wird das OCT-A eingesetzt, dessen verarbeitete Bilder die bewegte Blutzellendichte (häufig auch als Kapillardichte bezeichnet) beschreiben. Als Messkriterien werden analog zu den bisher beschriebenen Kriterien Pulsänderungen oder Änderungen der OCT-Signale, wie Änderungen der lokalen Blutzellengeschwindigkeit, des lokalen Blutflusses oder der Dichte der bewegten Blutzellen, genutzt, die dann den weiter oben definierten Blutdruckwerten zugeordnet werden.

### Bezugszeichenliste

- 1: Einheit zur Erzeugung und Applikation eines veränderbaren Stimulationsdruckes
- 1.1: Druckapplikator
- 1.2: Druckerzeugungseinheit
- 1.3: Halterung
- 2: Bildgebungseinheit
- 3: Tonometer
- 4: Rechen- und Steuereinheit
- 5: Ein- und Ausgabeeinheit
- 6: digitaler Videorekorder
- 7: Ergebnisspeichereinheit
- 8: Daten- und Bildauswerteeinheit
- 9: Signalanalyseeinheit
- 10: Einheit zur Bildung von spektralen Quotientensignalen
- 11: Einheit zur Bildung von Gefäßdurchmessersignalen

- A: Auge
- U: Untersucher
- B: Messbereich
- s(t): Zeitsignal

- rPP: retinaler Perfusionsdruck(wert)
- rP: retinaler Blutdruck(wert)
- rPaₖᵣᵢₜ: retinaler arterieller kritischer Blutdruck(wert)
- rPa: retinaler arterieller Blutdruck
- rPv: retinaler venöser Blutdruck(wert) (innerhalb des Augapfels)
- RVP: retinaler venöser Blutdruck(wert) außerhalb des Augapfels
- SD: (veränderbarer) Stimulationsdruck(wert)
- IOP: Intraokulardruck(wert)
- IOP₀: Ruhe-Intraokulardruck(wert)
- rPa_{VS}: retinaler arterieller Verschlussdruck(wert) (außerhalb des Augapfels)

## Patentansprüche

1. Vorrichtung zur Bestimmung globaler und lokaler retinaler Blutdruckwerte (rP) an einem Auge (A) eines Patienten, enthaltend eine auf das Auge (A) wirkende Einheit zur Erzeugung und Applikation eines veränderbaren Stimulationsdruckes (1), eine Bildgebungseinheit (2),
ein Tonometer (3), eine Rechen- und Steuereinheit (4) mit einer Ein- und Ausgabeeinheit (5), die mit der Einheit zur Erzeugung und Applikation eines veränderbaren Stimulationsdruckes (1) verbunden ist, wobei die Einheit zur Erzeugung und Applikation eines veränderbaren Stimulationsdruckes (1) einen Druckapplikator (1.1) enthält, der am Kopf des Patienten ortsfest zum Auge (A) außerhalb der Hornhaut und eines Lichtweges der Bildgebungseinheit (2) druckfrei flächig an das Auge (A) anlegbar ist, **dadurch gekennzeichnet,**
**dass** das Tonometer (3) dazu ausgelegt ist, einen sich in Abhängigkeit von einem applizierten veränderbaren Stimulationsdruck (SD) ändernden Intraokulardruck (IOP) zu messen und, dass
- in einer ersten Alternative zum manuellen Messen eines Intraokulardruckes (IOP) die Bildgebungseinheit (2) eine Spaltlampe mit einer Hruby-Linse ist und das Tonometer (3) ein Applanationstonometer ist, wobei die Spaltlampe so konfiguriert ist, dass die Hruby-Linse und das Applanationstonometer alternativ an der Spaltlampe anbringbar sind, sodass entweder eine Bildgebung oder eine Messung des Intraokulardruckes (IOP) während eines anliegenden Stimulationsdruckes (SD) möglich ist, oder
- in einer zweiten Alternative zum automatischen Messen eines Intraokulardruckes (IOP), die Bildgebungseinheit (2) eine modifizierte Netzhautkamera umfasst und das Tonometer (3) ein Rebound-Tonometer oder ein Non-Contact-Tonometer ist und in der Bildgebungseinheit (2) integriert ist, wobei die modifizierte Netzhautkamera und das Rebound-Tonometer oder das Non-Contact-Tonometer zeitgleich auf das Auge (A) eingestellt sind, sodass eine Bildgebung und die Messung des Intraokulardruckes (IOP) zeitgleich, während des anliegenden Stimulationsdruckes (SD), möglich ist.

2. Vorrichtung nach Anspruch 1, **dadurch gekennzeichnet,**
**dass** die Einheit zur Erzeugung und Applikation eines veränderbaren Stimulationsdruckes (1) so ansteuerbar ist, dass der applizierte veränderbare Stimulationsdruck (SD) in Richtung und Geschwindigkeit eines Anstieges änderbar ist sowie konstant gehalten werden kann, und die Ein- und Ausgabeeinheit (5) dazu ausgelegt ist, dass die Ansteuerung durch einen Untersucher (U) über die Ein- und Ausgabeeinheit (5) erfolgt.

3. Vorrichtung nach Anspruch 2 sofern rückbezogen auf Anspruch 1, zweite Alternative, **dadurch gekennzeichnet,**
**dass** die Bildgebungseinheit (2) einen digitalen Bildsensor oder eine bildgebende Einheit auf Basis der optischen Kohärenztomografie oder der Laser-Scanning-Technik aufweist und ein digitaler Videorekorder (6) vorhanden ist, der mit der Bildgebungseinheit (2) und der Rechen- und Steuereinheit (4) verbunden ist, und die Ein- und Ausgabeeinheit (5) einen Monitor aufweist und dazu ausgelegt ist, dass der Untersucher (U) wahlweise online Bilder der Bildgebungseinheit (2) oder vom digitalen Videorekorder (6) aufgezeichnete Videosequenzen der Bilder ansehen und zur Untersuchung einsetzen kann.

4. Vorrichtung nach Anspruch 3, **dadurch gekennzeichnet,**
**dass** eine Daten- und Bildauswerteeinheit (8) vorhanden ist, die mit dem digitalen Videorekorder (6), der Bildgebungseinheit (2), der Rechen- und Steuereinheit (4) und der Ein- und Ausgabeeinheit (5) in Verbindung steht, und die Ein- und Ausgabeeinheit (5) dazu ausgelegt ist, dass der Untersucher (U) in am Monitor angezeigten Bildern der Bildgebungseinheit (2) oder Bildern der Videosequenzen des digitalen Videorekorders (6) Messorte für erkannte visuelle Messkriterien festlegen kann und die Koordinaten der Messorte gemeinsam mit den visuellen Messkriterien, jeweils einem retinalen Blutdruckwert (rP) zugeordnet, abspeichern und in einem Druckmappingbild eintragen kann.

5. Vorrichtung nach Anspruch 3, **dadurch gekennzeichnet,**
**dass** die Einheit zur Erzeugung und Applikation eines veränderbaren Stimulationsdruckes (1) einen Drucksensor enthält, um jeweils einem gemessenen Intraokulardruckwert (IOP) bzw. jedem Bild der Videosequenz einen Stimulationsdruckwert (SD) zuordnen zu können.

6. Vorrichtung nach Anspruch 1, zweite Alternative, **dadurch gekennzeichnet,**
**dass** die Bildgebungseinheit (2) einen digitalen Bildsensor zur Erzeugung einer Videosequenz aufweist und eine Daten- und Bildauswerteeinheit (8) sowie eine Signalanalyseeinheit (9) vorhanden sind, wobei die Daten- und Bildauswerteeinheit (8) dazu ausgelegt ist, eine bewegungskorrigierte Videosequenz von der Netzhaut des Auges (A) zu erzeugen, und für jedes Pixel oder für eine beliebige Pixelgeometrie von Bildern der Videosequenz ein Signal bildet und einem Zeitsignal (s(t)) der Rechen- und Steuereinheit (4) zuordnet.

7. Vorrichtung nach Anspruch 6, **dadurch gekennzeichnet,**
**dass** die modifizierte Netzhautkamera aus mindestens zwei Farbkanälen und einem doppelbandigen Bandpassfilter besteht und eine Einheit zur Bildung von spektralen Quotientensignalen (10) vorhanden ist, die für jedes Pixel oder eine zusammenfassende Pixelgeometrie der Bilder der Videosequenz ein beleuchtungsunabhängiges spektrales Quotientensignal bildet und es einem Zeitsignal (s(t)) der Rechen- und Steuereinheit (4) zuordnet.

8. Vorrichtung nach Anspruch 1, zweite Alternative, **dadurch gekennzeichnet,**
**dass** eine Einheit zur Bildung von Gefäßdurchmessersignalen (11) vorhanden ist, die für jedes Gefäßsegment ein mit einem Durchmesser korrelierendes Gefäßdurchmessersignal bildet und dieses einem Zeitsignal (s(t)) der Rechen- und Steuereinheit (4) zugeordnet.

## Claims

1. Device for determining global and local retinal blood pressure values (rP) in a patient's eye (A), comprising a unit acting on the eye (A) for generating and applying a variable stimulation pressure (1), an imaging unit (2), a tonometer (3), a computing and control unit (4) with an input and output unit (5) connected to the unit for generating and applying a variable stimulation pressure (1), wherein the unit for generating and applying a variable stimulation pressure (1) contains a pressure applicator (1.1) that can be applied to the patient's eye (A) in a pressure-free flat manner at the head of the patient, fixed in position relative to the eye (A), outside the cornea and outside the light path of the imaging unit (2), **characterized in that** the tonometer (3) is designed to measure intraocular pressure (IOP) that changes in response to an applied variable stimulation pressure (SD), and that
- in a first alternative to manually measuring intraocular pressure (IOP) , the imaging unit (2) is a slit lamp with a Hruby lens and the tonometer (3) is an applanation tonometer, wherein the slit lamp is configured such that the Hruby lens and the applanation tonometer can be alternatively attached to the slit lamp, so that either imaging or measurement of the intraocular pressure (IOP) is possible during an applied stimulation pressure (SD), or
- in a second alternative for automatically measuring intraocular pressure (IOP), the imaging unit (2) comprises a modified retinal camera and the tonometer (3) is a rebound tonometer or a non-contact tonometer and is integrated into the imaging unit (2), wherein the modified retinal camera and the rebound tonometer or the non-contact tonometer are simultaneously adjusted to the eye (A), so that imaging and measurement of the intraocular pressure (IOP) are possible simultaneously during the applied stimulation pressure (SD).

2. Device according to claim 1, **characterized in that**
the unit for generating and applying a variable stimulation pressure (1) can be controlled in such a way that the applied variable stimulation pressure (SD) can be changed in terms of direction and speed of increase and can also be kept constant, and the input and output unit (5) is designed so that control is carried out by an examiner (U) via the input and output unit (5).

3. Device according to claim 2, referring back to claim 1, second alternative, **characterized in that**
the imaging unit (2) has a digital image sensor or an imaging unit based on optical coherence tomography or laser scanning technology, and a digital video recorder (6) is provided which is connected to the imaging unit (2) and the computing and control unit (4), and the input and output unit (5) has a monitor and is designed so that the examiner (U) can optionally view images from the imaging unit (2) online or video sequences of the images recorded by the digital video recorder (6) and use them for examination.

4. Device according to claim 3, **characterized in that**
a data and image evaluation unit (8) is provided, which is connected to the digital video recorder (6), the imaging unit (2), the computing and control unit (4), and the input and output unit (5), and the input and output unit (5) is designed so that the examiner (U) can specify measurement locations for recognized visual measurement criteria in images from the imaging unit (2) or images from the video sequences of the digital video recorder (6) displayed on the monitor, and can store the coordinates of the measurement locations together with the visual measurement criteria, each assigned to a retinal blood pressure value (rP), and enter them in a pressure mapping image.

5. Device according to claim 3, **characterized in that**
the unit for generating and applying a variable stimulation pressure (1) contains a pressure sensor to be able to assign a stimulation pressure value (SD) to each measured intraocular pressure value (IOP) or each image of the video sequence.

6. Device according to claim 1, second alternative, **characterized in that**
the imaging unit (2) has a digital image sensor for generating a video sequence and a data and image evaluation unit (8) and a signal analysis unit (9) are provided, wherein the data and image evaluation unit (8) is designed to generate a motioncorrected video sequence from the retina of the eye (A), and forms a signal for each pixel or for any pixel geometry of images of the video sequence and assigns it to a time signal (s(t)) of the computing and control unit (4).

7. Device according to claim 6, **characterized in that**
the modified retinal camera consists of at least two color channels and a dual-band bandpass filter, and that there is a unit for forming spectral quotient signals (10) which forms an illumination-independent spectral quotient signal for each pixel or a summary pixel geometry of the images of the video sequence and assigns it to a time signal (s(t)) of the computing and control unit (4).

8. Device according to claim 1, second alternative, **characterized in that**
a unit for forming vessel diameter signals (11) is provided, which forms a vessel diameter signal correlated with a diameter for each vessel segment and assigns it to a time signal (s(t)) of the computing and control unit (4).

## Revendications

1. Dispositif pour déterminer les valeurs globales et locales de la pression sanguine rétinienne (rP) dans un œil (A) d'un patient , comprenant une unité agissant sur l'œil (A) pour générer et appliquer une pression de stimulation variable (1), une unité d'imagerie (2),
un tonomètre (3), une unité de calcul et de commande (4) avec une unité d'entrée et de sortie (5) qui est reliée à l'unité destinée à générer et à appliquer une pression de stimulation variable (1), l'unité destinée à générer et à appliquer une pression de stimulation variable (1) comprenant un applicateur de pression (1.1) qui peut être appliqué de manière fixe sur la tête du patient, à l'œil (A), à l'extérieur de la cornée et d'un trajet lumineux de l'unité d'imagerie (2), sans pression et à plat sur l'œil (A), **caractérisé en ce que**
que le tonomètre (3) est conçu pour mesurer une pression intraoculaire (PIO) qui varie en fonction d'une pression de stimulation variable (PS) appliquée et que
- dans une première alternative à la mesure manuelle d'une pression intraoculaire (IOP) , l'unité d'imagerie (2) est une lampe à fente avec une lentille de Hruby et le tonomètre (3) est un tonomètre à aplanation, la lampe à fente étant configurée de telle sorte que la lentille de Hruby et le tonomètre à aplanation peuvent être fixés en alternance à la lampe à fente , de sorte qu'il est possible soit d'effectuer une imagerie, soit de mesurer la pression intraoculaire (PIO) pendant une pression de stimulation (PS) appliquée, ou
- dans une deuxième alternative pour mesurer automatiquement une pression intraoculaire (PIO), l'unité d'imagerie (2) comprend une caméra rétinienne modifiée et le tonomètre (3) est un tonomètre à rebond ou un tonomètre sans contact et est intégré dans l'unité d'imagerie (2), la caméra rétinienne modifiée et le tonomètre à rebond ou le tonomètre sans contact étant réglés simultanément sur l'œil (A) , de sorte qu'une imagerie et la mesure de la pression intraoculaire (PIO) sont possibles simultanément, pendant la pression de stimulation appliquée (SD).

2. Dispositif selon la revendication 1, **caractérisé en ce que**
l'unité de génération et d'application d'une pression de stimulation variable (1) peut être commandée de telle sorte que la pression de stimulation variable appliquée (SD) peut être modifiée en termes de direction et de vitesse d'augmentation et peut être maintenue constante, et l'unité d'entrée et de sortie (5) est conçue de telle sorte que la commande s'effectue par un examinateur (U) via l'unité d'entrée et de sortie (5).

3. Dispositif selon la revendication 2, dans la mesure où il se réfère à la revendication 1, deuxième alternative, **caractérisé en ce que**
l'unité d'imagerie (2) comporte un capteur d'images numérique ou une unité d'imagerie basée sur la tomographie par cohérence optique ou la technique de balayage laser et qu'il existe un enregistreur vidéo numérique (6) qui est relié à l'unité d'imagerie (2) et à l'unité de calcul et de commande (4), et l'unité d'entrée et de sortie (5) comprend un moniteur et est conçue de manière à ce que l'examinateur (U) puisse choisir de visualiser en ligne les images de l'unité d'imagerie (2) ou les séquences vidéo des images enregistrées par l'enregistreur vidéo numérique (6) et les utiliser pour l'examen.

4. Dispositif selon la revendication 3, **caractérisé en ce**
**qu'**il existe une unité d'évaluation des données et des images (8) qui est reliée à l'enregistreur vidéo numérique (6), à l'unité d'imagerie (2), l'unité de calcul et de commande (4) et l'unité d'entrée et de sortie (5), et l'unité d'entrée et de sortie (5) est conçue de manière à ce que l'examinateur (U) puisse, dans les images de l'unité d'imagerie (2) ou les images des séquences vidéo de l'enregistreur vidéo numérique (6) affichées sur le moniteur (6), et l'unité d'entrée et de sortie (5) est conçue pour que l'examinateur (U) puisse définir des emplacements de mesure pour des critères de mesure visuels reconnus dans les images de l'unité d'imagerie (2) ou les images des séquences vidéo de l'enregistreur vidéo numérique (6), et enregistrer les coordonnées des emplacements de mesure avec les critères de mesure visuels, attribués à une valeur de pression sanguine rétinienne (rP) respective, et les inscrire dans une image de cartographie de pression.

5. Dispositif selon la revendication 3, **caractérisé en ce que**
l'unité de génération et d'application d'une pression de stimulation variable (1) comprend un capteur de pression afin de pouvoir attribuer une valeur de pression de stimulation (SD) à chaque valeur de pression intraoculaire (IOP) mesurée ou à chaque image de la séquence vidéo.

6. Dispositif selon la revendication 1, deuxième alternative, **caractérisé en ce que**
l'unité d'imagerie (2) comporte un capteur d'image numérique pour générer une séquence vidéo et qu'il existe une unité d'évaluation des données et des images (8) ainsi qu'une unité d'analyse des signaux (9), l'unité d'évaluation des données et des images (8) étant conçue pour générer une séquence vidéo corrigée en fonction des mouvements à partir de la rétine de l'œil (A), et forme un signal pour chaque pixel ou pour une géométrie de pixels quelconque d'images de la séquence vidéo et l'attribue à un signal temporel (s(t)) de l'unité de calcul et de commande (4).

7. Dispositif selon la revendication 6, **caractérisé en ce que**
la caméra rétinienne modifiée se compose d'au moins deux canaux de couleur et d'un filtre passe-bande à double bande et qu'il existe une unité pour former des signaux de quotient spectral (10) qui, pour chaque pixel ou une géométrie de pixels récapitulative des images de la séquence vidéo, forme un signal de quotient spectral indépendant de l'éclairage et l'attribue à un signal temporel (s(t)) de l'unité de calcul et de commande (4).

8. Dispositif selon la revendication 1, deuxième alternative, **caractérisé en ce**
**qu'**il existe une unité pour la formation de signaux de diamètre de vaisseau (11) qui forme pour chaque segment de vaisseau un signal de diamètre de vaisseau corrélé à un diamètre et l'associe à un signal temporel (s(t)) de l'unité de calcul et de commande (4).
